# FASCICULE DE BREVET EUROPEEN

(11) **EP 3 773 682 B1**
(45) Date de publication et mention de la délivrance du brevet: **13.09.2023**
(21) Numéro de dépôt: 19712611.3
(22) Date de dépôt: 28.03.2019
(51) Int. Cl.: A61P 5/08, A61M 5/315, A61K 38/25

(54) **PRÉSENTATIONS INJECTABLES, SERINGUES ET COMPOSITIONS À LIBÉRATION PROLONGÉE ET/OU CONTROLÉE DE LANRÉOTIDE**
INJIZIERBARE DARREICHUNGSFORMEN, SPRITZEN UND ZUSAMMENSETZUNGEN MIT VERZÖGERTER UND/ODER KONTROLLIERTER FREISETZUNG VON LANREOTID
INJECTABLE PRESENTATIONS, SYRINGES AND COMPOSITIONS WITH SUSTAINED AND/OR CONTROLLED RELEASE OF LANREOTIDE

(30) Priorité: 28.03.2018 FR 1852662
(43) Date de publication de la demande: 17.02.2021
(73) Titulaire: Edix SA, 2320 Luxembourg (LU)
(72) Inventeur: GONZALEZ GARCIA, Maria Isabel, 08880 Cubelles (ES); ROCA TORRELLAS, José Maria, 08029 Barcelona (ES); BOURGOIS, Tabatha, 08025 Barcelona (ES); LACHAMP, Laurence, 08850 Gava Barcelona (ES); LACOMBE, Frederic, 08173 Sant Cugat del Valles (ES)
(74) Mandataire: Bourgouin, André
(86) Numéro de dépôt international: PCT/EP2019/057845
(87) Numéro de publication internationale: WO 2019/185786

(56) Documents cités:
- WO-A1-2016/022831
- WO-A2-96/07398
- WO-A2-2011/085957
- US-A1- 2012 156 259
- US-A1- 2015 374 929

## Description

La présente demande a pour objet de nouvelles présentations injectables pour compositions pharmaceutiques destinées à la libération prolongée et/ou contrôlée de lanréotide.

Plus particulièrement la présente demande a pour objet de nouvelles présentations injectables, kits ou seringues comprenant une composition à libération prolongée ou contrôlée de lanréotide ou de l'un de ses sels.

Le lanréotide commercialisé et souvent utilisé sous forme d'acétate est un octapeptide analogue de la somatostatine naturelle. Comme la somatostatine, le lanréotide est un inhibiteur de différentes fonctions endocrines, neuro-endocrines, exocrines et paracrines. Il présente une forte affinité pour les récepteurs à la somatostatine humaine (SSTR) 2 et 5, et une affinité faible sur les SSTR 1, 3 et 4. L'inhibition de l'hormone de croissance s'expliquerait principalement par cette activité au niveau des récepteurs SSTR 2 et 5. Le lanréotide est plus actif que la somatostatine naturelle et présente une durée d'action plus longue. Sa sélectivité marquée vis-à-vis de la sécrétion de l'hormone de croissance par rapport à celle de l'insuline en a d'abord fait un produit avec des compositions adaptées au traitement de l'acromégalie.

Le principal domaine d'application du lanréotide est aujourd'hui celui des traitements des tumeurs neuroendocrines (NET), d'abord les symptômes des syndromes carcinoïdes puis le contrôle de ces tumeurs grâce à son activité antiproliférative, pour lesquelles on a jusqu'à présent utilisé les compositions mises au point pour traiter l'acromégalie. Ces compositions permettent de délivrer des doses faibles de façon continue pendant des années.

En ce qui concerne le traitement des tumeurs neuroendocrines, il est important de pouvoir adapter rapidement chaque traitement à chaque évolution de la maladie, mais aussi de pouvoir facilement administrer des doses plus élevées dont on connaît les bénéfices pour le traitement des syndromes carcinoïdes car ceci permet de mieux contrôler l'évolution de ces tumeurs sur des périodes plus longues. Cela serait plus facile avec des produits qui donnent immédiatement les niveaux nécessaires et dont le relargage puisse être interrompu plus rapidement, que la durée d'administration soit de plusieurs semaines ou de plusieurs mois. Il serait également souhaitable que ce but soit obtenu avec des produits plus faciles et plus confortables à injecter.

Des compositions du lanréotide sont décrites dans la demande de brevet WO9607398 qui mentionne des formes solides ou semi-solides de peptides gélifiables dont le lanréotide, qui vont gélifier pour donner un relargage prolongé du lanréotide.

Il est aussi fait mention de suspensions ou compositions semi-solides, décrites comme hautement visqueuses ou pâteuses, dans lesquelles on ajoute un solvant pour obtenir la consistance semi-solide qui forme automatiquement un gel après interaction avec les fluides corporels. Le relargage contrôlé de la composition de lanréotide est décrit comme dépendant fortement de la viscosité et du volume du produit injecté. Ces semi-solides sont aussi décrits comme des rods ou « barres » d'extrusion, en relation avec l'importance accordée à préserver la forme du produit injecté et l'obligation précisée d'injecter le produit avec une aiguille de gros diamètre. La quantité de solvant (eau) est précisée comme devant être limitée de façon à éviter tous risques de solubilisation du lanréotide gélifiable, afin de préserver un dépôt résistant, qui gélifiera au site d'injection.

Le document WO 9607398 explique également l'importance accordée à la forme et au volume du dépôt. Il est montré qu'en réduisant le diamètre du dépôt de lanréotide lors de l'injection, on observe un contrôle du relargage in-vitro beaucoup plus variable. Les compositions trop diluées sont considérées comme indésirables parce qu'elles se dispersent après injection et ne forment pas le gel responsable de la libération prolongée du produit.

Le document WO2015004125 décrit une composition pharmaceutique pour une durée d'action prolongée d'au moins deux mois, comprenant le lanréotide comme ingrédient actif à une concentration allant de 35 à 55% en poids par rapport au poids total de la composition, un co-solvant choisi parmi les polymères hydrosolubles de bas poids moléculaire tels que la polyvinylpyrrolidone (PVP) et le polyéthylène glycol (PEG), la N-méthyl-2-pyrrolidone (NMP), la N-éthyl-2-pyrrolidone (NEP), le propylène glycol, le glycérol, le glycofurol, l'éthanol, l'alcool benzylique et un mélange de ceux-ci, et de l'eau.

La demande de brevet américain US 2015/374929 décrit des dispositifs qui comprennent une partie pénétrante formée de deux cylindres creux concentriques, le cylindre creux externe comprenant une pluralité de pores en communication fluidique avec la partie réservoir de fluide, le cylindre creux interne définissant une lumière en communication fluidique avec une seconde partie du réservoir de fluide.

La demande de brevet américain US 2012/156259 décrit des hydrogels insolubles dans l'eau à base de polyéthylène glycol, qui comprennent des fractions de squelette interconnectées par des liaisons dégradables par hydrolyse, lesdites fractions comprenant de plus des groupes fonctionnels réactifs.

La demande de brevet international WO 2016/022831 concerne des seringues, des kits contenant celles-ci, et des procédés associés. Certaines seringues sont préchargées avec une pâte et ont un corps de seringue définissant un réservoir, une pâte disposée à l'intérieur du réservoir, la pâte ayant une concentration de matières solides de plus de 50 mg/ml.

La demande de brevet international WO 2011/085957 concerne un procédé pour la préparation de compositions pharmaceutiques injectables pour la libération prolongée d'analogues de somatostatine et des compositions pharmaceutiques préparées selon le procédé.

Le lanréotide est commercialisé et utilisé dans des produits tels que Somatuline^{®} Autogel^{®}, Somatuline L.P. ou Somatuline^{®} Depot. Le produit Somatuline L.P. 120 mg, par exemple se présente sous forme d'une seringue spécifique pré-remplie de 0,5 mL comportant un système de sécurité automatique et une aiguille spécifique pré-montée de 1,20 mm x 20 mm. Les compositions commercialisées renferment de l'acétate de lanréotide qui se présente sous la forme d'un produit pâteux d'aspect blanchâtre et translucide qui doit être injecté dans le quadrant supéro-externe du fessier, en enfonçant l'aiguille dans toute sa longueur (voie sous-cutanée profonde). Par la suite et dans un soucis de simplification, par « Somatuline^{®} » s'entendra à titre non limitatif « Somatuline^{®} Autogel^{®} », « Somatuline L.P », « Somatuline^{®} Depot » ou tout autre nom comercial donné au produit.

Les compositions commercialisées ont été formulées pour obtenir après injection de l'acétate de lanréotide une forme et un volume de dépôt menant à une gélification in-situ au contact des fluides corporels. Pour obtenir cet effet, il est nécessaire d'utiliser des aiguilles de diamètre interne d'au moins 1,00 mm pour obtenir un dépôt compact. Ce type d'aiguille permet l'injection de produits semi-solides, pâteux ou très visqueux.

Les compositions commercialisées existantes à ce jour contiennent une concentration de lanréotide de 25% en poids. Pour pouvoir injecter ce produit pâteux, il est nécessaire d'utiliser une seringue spéciale de faible diamètre interne d'environ 3,00 mm et d'une longueur de 80 mm. Le produit injecté ne peut être que d'un volume maximum de 0,5 mL. Toutefois la finesse et la longueur de la seringue posent des problèmes pour implanter une grosse aiguille et injecter confortablement et précisément ce produit très visqueux.

Par ailleurs, il peut être noté que les aiguilles de diamètre interne d'au moins 1mm sont également responsables de problèmes de douleur au site d'injection ou à la suite de la formation de nodules. Une autre contrainte, liée à la consistance pâteuse du lanréotide, rencontrée avec une composition de lanréotide de 25% en poids est l'impossibilité d'injecter le produit par voie intramusculaire à cause de la force d'injection trop grande associée à la perte de charge d'une aiguille d'au moins 25 mm de longueur. Il n'est pas non plus souhaitable d'injecter le lanréotide d'une telle composition par voie sous-cutanée superficielle à cause du diamètre trop important de l'aiguille et de la faible tolérance locale d'un dépôt peu déformable.

Les compositions commercialisées à base de lanréotide sont utilisées pour une administration par voie sous-cutanée profonde, sur des périodes pouvant varier de semaines à une fois par mois ou une fois tous les 2 mois chez certains patients, pour des traitements pouvant s'étendre sur plusieurs années.

Avec les aiguilles actuelles de 20 mm de longueur, cette voie sous-cutanée profonde risque parfois d'être intramusculaire ou à la frontière des deux tissus. Il serait donc utile de disposer d'une présentation injectable et d'une composition qui permette de choisir avec plus de précision la route d'injection. Dans certains cas il peut aussi être souhaitable de préférer la voie intramusculaire, par exemple pour éviter de possibles risques de tolérance locale ou de réponse immunitaire lié au principe actif.

Il existe donc un important besoin de simplifier les injections, de les rendre moins douloureuses et de pouvoir changer le type et la localisation des dépôts pour éviter les problèmes de nodules. Il y a aussi un besoin d'améliorer les profils de relargages du lanréotide pour couvrir les besoins en fin d'intervalle avant réinjection dans le cas de l'acromégalie, ou pour augmenter les niveaux et mieux contrôler les profils de relargage dans le cas des tumeurs neuroendocrines. Il est clair qu'il existe un besoin médical non satisfait auquel il est souhaitable d'apporter des réponses en proposant de nouvelles compositions dont l'administration puisse reposer sur des dispositifs simples voir standards, du type seringue munie d'un piston actionné manuellement.

Depuis le début du développement des compositions à libération prolongée du lanréotide actuellement commercialisées, la composition retenue pour faire face aux besoins de doses plus élevées de lanréotide a consisté à rapprocher les injections ou à les multiplier au même moment mais sans résoudre de manière satisfaisante les problèmes liés à ces injections.

En effet, l'utilisation d'un dispositif manuel du type seringue standard avec une aiguille plus fine combinée avec une composition moins visqueuse de lanréotide n'a jamais été envisagé car cette composition allait à l'encontre d'un préjugé défavorable pour l'homme de l'art.

En effet, l'homme du métier du domaine des compositions pharmaceutiques sait qu'une diminution de la concentration en lanréotide, même de quelques pourcents, va avoir pour effet une diminution de la viscosité et donc une solubilisation plus rapide de la composition de lanréotide au contact des fluides corporels au niveau du site d'injection.

Au vu de l'enseignement de l'art antérieur et notamment de la demande WO 9607398 il est possible de s'attendre à ce que l'effet retard provoqué par la gélification du produit concentré ne se produise plus, entrainant un relargage rapide du produit.

Par ailleurs, la demande WO 9607398 préconise également l'utilisation d'une aiguille d'un diamètre interne d'au moins 1,00 mm afin d'injecter le lanréotide pour que le dépôt du produit conserve un profil de relargage d'au moins 15 jours.

L'administration d'une composition de lanréotide présentant une concentration moins élevée que celle décrite dans cette demande avec une aiguille de moins de 1,00 mm va donc à l'encontre de l'enseignement de l'art antérieur sur deux points essentiels qui sont les caractéristiques de la présente demande.

De façon tout à fait inattendue, les auteurs de la présente demande ont mené des recherches et mis en évidence le fait qu'une composition de lanréotide moins visqueuse pouvait conduire à des dépôts qui sont différents des gels précédemment décrits mais qui sont aussi capables de donner un relargage contrôlé de lanréotide, lorsque ladite composition est injectée à l'aide d'une aiguille plus fine que celle utilisée pour les compositions commerciales. Cette découverte rend possible la réalisation de compositions de lanréotide diminuant ou supprimant les désavantages liés à la fabrication spécifique et à l'utilisation complexe des compositions actuellement commercialisées.

Les améliorations et simplifications apportées aux injections par cette demande peuvent ainsi permettre des présentations pré-remplies de compositions à libération prolongée et/ou contrôlée de lanréotide dans les réservoirs classiques des dispositifs d'injections standards que sont les seringues ou les cartouches habituelles en verre ou polymériques (ex. Cyclo Olefin Polymers ou COC cyclic olefin copolymer). Ces améliorations sont telles qu'elles permettent l'utilisation de réservoirs de tailles et diamètres classiques de ces présentations pré-remplies qui n'étaient pas compatibles avec les besoins d'injection prolongée et/ou contrôlée et les compositions actuelles dont les présentations commerciales nécessitent des techniques spécifiques de remplissage dans des réservoirs de faibles diamètres combinés avec des aiguilles de gros diamètre, ou des dispositifs d'injection particuliers qui permettent de réaliser des injections de dépôts à libération prolongée ou contrôlée.

### DESCRIPTION DE L'INVENTION

Ainsi, la présente demande consiste à fournir de nouvelles compositions injectables à base de lanréotide ou de l'un de ses dérivés, tels que ses sels permettant:
- de réduire les problèmes d'injection tels que les difficultés d'implantation des aiguilles de diamètre interne d'au moins 1,00 mm,
- de réduire les effets indésirables au site d'injection tels que l'apparition de nodules,
- d'enrichir la gamme de traitement en proposant par exemple des administrations intramusculaires, intradermiques, intraoculaires ou encore sous-cutanées superficielles.

La présente invention concerne une seringue pré-remplie caractérisée en ce que son diamètre est supérieur à 3,00 mm, préférentiellement compris entre 4,00 mm et 7,00 mm, et elle est équipée d'une aiguille ayant une longueur comprise de préférence entre 10 et 40 mm et elle contient une composition pharmaceutique comprenant :
1) un solvant aqueux constitué d'eau et éventuellement d'un solvant organique miscible à l'eau, de préférence un alcool, encore plus de préférence l'éthanol,
2) du lanréotide ou l'un de ses sels, de préférence l'acétate de lanréotide à une concentration allant de 11 à 25% en poids de lanréotide, de préférence de 18 à 25 %, en poids de lanréotide par rapport au poids total de la composition,
3) éventuellement un ou plusieurs acides aminés, de préférence l'arginine,
4) éventuellement un excipient monomérique accepté par la pharmacopée caractérisée en ce que ladite seringue est équipée d'une aiguille standard ayant un diamètre externe égal ou inférieur à 1,00 mm et un diamètre interne égal ou inférieur à 0,80 mm, le diamètre de ces aiguilles permettant de réduire les difficultés d'implantation des aiguilles de diamètre interne d'au moins 1,00 mm.

Notamment lorsque des seringues de type HyPak^{™}, HyLok^{™} ou des seringues en plastiques en COP de type Clearject^{®}, sont utilisées, le diamètre interne des seringues est plus préférentiellement supérieur à 4,00 mm jusqu'à 7,00 mm de diamètre .

Selon un mode préféré de la demande l'excipient monomérique est au moins un composé choisi parmi le lactose, le dextrose, le sucrose, le mannitol, le sorbitol, le glucose, le fructose, le mannose, le galactose, l'arabinose, le fucose, l'acide citrique ou un de ses sels ou esters, l'acide lactique ou un de ses sels ou esters, l'acide acétique ou un des ses sels ou esters et leurs mélanges et la composition pharmaceutique selon la présente demande ne contient pas d'excipient polymérique tel que l'acide (d,l) polylactique (PLA), un copolymère d'acide lactique et d'acide glycolique (PLGA), le polyphénylène propylène, un copolymère d'éthylène et d'acétate de vinyle; un copolymère d'acide gras et d'acide sébacique, un copolymère de poly (acide érucique dimère-acide sébacique), un copolymère poly (acide fumarique-acide sébacique), la chitine, le collagène, la gélatine, le monométhyl polyéthylène glycol / acide polylactique, des copolymères monométhyl polyéthylène glycol / acide polylactique, polyéthylène glycol / acide polylactique ou un copolymère polyéthylène glycol / acide polylactique.

Selon un mode préféré, la présente demande concerne une seringue manuelle avec tige de piston munie d'une aiguille standard du type luer-lock ayant un diamètre externe de 0,80 à 1,00 mm, un diamètre intérieur et 0,60 à 0,80 mm et une longueur d'environ 20 mm, pré-remplie avec une composition pharmaceutique à libération prolongée pour administration parentérale, ladite composition comprenant un solvant aqueux constitué d'eau et éventuellement d'alcool éthylique à 3%, de l'acétate de lanréotide en quantité suffisante pour que la concentration en lanréotide soit d'environ de préférence de 18 à 25 %, plus préférentiellement de 18 à 22%, plus préférentiellement encore de 18 à 21% en poids par rapport au poids total de la composition, et optionnellement de l'arginine à une concentration allant de 0,1 à 13% en poids par rapport au poids total de la composition.

Plus particulièrement, la demande a pour objet une seringue manuelle avec tige de piston munie d'une aiguille standard du type luer-lock ayant un diamètre externe d'environ 0,80 mm, un diamètre intérieur d'environ 0,60 mm et une longueur d'environ 20 mm, pré-remplie avec une composition ou d'une suspension pharmaceutique à libération prolongée pour administration parentérale, ladite composition comprenant de l'eau et de l'acétate de lanréotide en quantité suffisante pour que la concentration en lanréotide soit d'environ 20% en poids par rapport à l'eau.

Selon un mode de réalisation de la demande, un kit comprend une composition pharmaceutique telle que décrite ci-dessus et une seringue dont l'aiguille possède un diamètre externe égal ou inférieur à 1,00 mm, un diamètre interne égal ou inférieur à 0,8 mm et une longueur comprise de préférence entre 10 et 40mm. L'injection est réalisée en actionnant manuellement la tige de piston le long de la seringue.

Selon un mode de réalisation préféré, la présente demande concerne également une seringue pré-remplie, ladite seringue contenant la composition pharmaceutique telle que définie ci-dessus pour une libération prolongée d'un principe actif pendant au moins 1 mois.

Selon un autre mode de réalisation de la demande la seringue est une seringue manuelle standard de type luer-lock avec tige de piston standard munie d'une aiguille pouvant être standard, ladite aiguille ayant un diamètre externe de 0,90 à 1,00 mm, un diamètre intérieur de 0,70 à 0,80 mm et une longueur de 20 à 30 mm.

Selon un autre mode de réalisation préféré de la demande la seringue manuelle standard de type luer-lock comprenant une tige de piston standard munie d'une aiguille pouvant être standard, ladite aiguille ayant un diamètre externe de 0,90 mm, un diamètre intérieur de 0,70 mm et une longueur de 20 mm, pré-remplie avec une composition pharmaceutiqueà libération prolongée pour administration parentérale, ladite composition comprenant un solvant aqueux constitué d'eau et d'alcool éthylique à 3% en poids par rapport au poids total de la composition, de lanréotide d'une concentration de préférence de 18 à 25 %, plus préférentiellement de 18 à 22%, plus préférentiellement encore de 18 à 21% en poids par rapport au poids total de la composition et de l'arginine de 0,1 à 13% en poids par rapport au poids total de la composition.

Selon un autre mode de réalisation préféré de la demande la seringue manuelle comprend une tige de piston munie d'une aiguille standard du type luer-lock ayant un diamètre externe d'environ 0,80 mm, un diamètre intérieur d'environ 0,60 mm et une longueur d'environ 20 mm, pré-remplie avec une composition pharmaceutique à libération prolongée pour administration parentérale, ladite composition comprenant de l'eau et de l'acétate de lanréotide à une concentration de 20% de lanréotide en poids par rapport au poids total de la composition.

Selon un mode de réalisation préféré, la présente composition est administrée par voie parentérale par injection sous-cutanée normale ou profonde, ou intramusculaire chez un patient.

Selon un mode de réalisation préféré, la présente composition est telle que l'administration parentérale par injection sous cutanée normale ou profonde, ou intramusculaire chez un patient de ladite composition à l'aide de ladite seringue libère le lanréotide ou le sel de lanréotide sur une durée d'au moins 7 jours, de 7 à 14 jours, de 14 à 28 jours, de préférence d'au moins 28 jours.

Selon un mode de réalisation préféré, la présente composition est sous une forme adaptée à une administration parentérale par injection sous cutanée, normale ou profonde, ou intramusculaire chez un patient de ladite composition à l'aide de ladite seringue libérant le lanréotide ou le sel de lanréotide sur une durée d'au moins 7 jours, de 7 à 14 jours, de 14 à 28 jours, de préférence d'au moins 28 jours.

Selon un mode de réalisation préféré, le lanréotide est sous forme de sel ou de base libre. Les sels de lanréotide utilisables pour la demande sont de préférence des sels pharmaceutiquement acceptables d'acides organiques, tels que ceux des acides acétique, phénylacétique, lactique, malique, pamoïque, ascorbique, succinique, benzoïque, méthanesulfonique ou toluènesulfonique, ou des sels pharmaceutiquement acceptables des acides inorganiques tels que ceux des acides chlorhydrique, bromhydrique, hydriodique, sulfurique ou phosphorique.

Selon un mode de réalisation préféré, le lanréotide est sous la forme d'acétate de lanréotide. Selon un autre mode de réalisation préféré, le lanréotide est sous forme de base libre.

Quelle que soit la forme de lanréotide, c'est-à-dire sous forme saline ou base libre, au sens de la présente demande, la quantité de lanréotide, exprimée par exemple en concentration ou en pourcentage dans la composition, désigne le lanréotide sous forme de base libre.

Avantageusement, le lanréotide est présent à une concentration de 18%, 18,5%, 19%, 19,5%, 20%, 20,5%,21%, 21,5% ,22%, 22,5%, 23%, 23,5%, 24%, 24,5%, ou 25% en poids par rapport au poids total de la composition.

Si l'on définit comme semi-solide injectable une composition qui forme après injection un extrudât solide manipulable, la composition pharmaceutique en tant que telle ou présente dans une seringue pré-remplie selon la présente demande est une composition visqueuse, mais non semi-solide. Cette composition peut être injectée à l'aide d'un dispositif standard d'injection tel qu'une seringue avec tige de piston actionnée manuellement, équipée d'une aiguille classique. Les dimensions préférées de la seringue et de l'aguille sont indiquées ci-dessus.

La viscosité et d'autres caractéristiques d'état physique des compositions peuvent être évaluées en tant qu'injectabilité, lorsque l'étape d'administration est évaluée. L'injectabilité peut être déterminée en effectuant des tests d'injection simulés et selon différentes méthodes physiques. L'injectabilité peut être rapportée comme force d'injection ou force d'injection de seringue (SIF).

Le terme "injectabilité" qui peut être déterminé en mesurant la force d'injection, se réfère à l'aptitude de la composition pour l'administration parentérale en utilisant un dispositif pour injection, comme une seringue ou un injecteur.

La SIF peut être déterminée à l'aide d'un dynamomètre (L1000R, Lloyd Instruments Ltd.) équipé d'une cellule calibrée (NLC 100N, Lloyd Instruments Ltd.). La composition pharmaceutique selon la présente demande peut être conditionnée dans une seringue dont le diamètre est supérieur à 3,00 mm, de préférence supérieur ou égal à 3,50 mm de diamètre, plus préférentiellement supérieur à 4,00 mm jusqu'à 7,00 mm de diamètre couplée à une aiguille de 20 mm de longueur pour un diamètre intérieur variant de 0,60 à 1,00 mm comparables aux aiguilles actuelles et testée pour son aptitude à l'injection lors d'une décharge simulée à une vitesse lente de 100 mm/min, où la force appliquée par l'instrument sur le piston et son déplacement sont enregistrés. L'injection simulée est effectuée verticalement dans l'air. La force d'injection maximale exprimée en newtons (N) est dérivée des données recueillies pendant la décharge. De tels exemples de forces d'injection sont détaillés dans l'Exemple 9.

Il est connu de l'homme de l'art qu'avec de telles aiguilles plus fines il n'est pas possible de pratiquer des injections manuelles de produits commerciaux de type Somatuline^{®} même avec des seringues plus fines de type Somatuline^{®}, et donc encore moins avec des seringues standard de plus grand diamètre interne.

Selon un mode de réalisation préféré, la composition pharmaceutique en tant que telle ou présente dans une seringue pré-remplie selon la présente demande présente une injectabilité définie par une SIF allant de 5, 6, 7, 8, 9, 10, 11, 12, 13 ou 14 N lorsqu'elle est testée avec la méthode définie ci-dessus.

Selon un mode de réalisation préféré, la composition pharmaceutique en tant que telle ou présente dans une seringue pré-remplie selon la présente demande présente une injectabilité définie par une SIF allant de 5 à 12 N ou de 5 à 14 N lorsqu'elle est testée avec la méthode définie ci-dessus et une aiguille de 1,00 mm de diamètre interne pour une composition contenant de préférence de 18 à 25 %, plus préférentiellement de 18 à 22%, plus préférentiellement encore de 18 à 21% de lanréotide.

De préférence, la SIF de la composition pharmaceutique en tant que telle ou présente dans une seringue pré-remplie selon la présente demande va de 5 à 6 N.

Selon un autre mode de réalisation préféré, la composition pharmaceutique en tant que telle ou présente dans une seringue pré-remplie selon la présente demande présente une injectabilité définie par une SIF allant de 7 à 12 N ou de 5 à 14 N lorsqu'elle est testée avec la méthode définie ci-dessus et une aiguille de 0,80mm de diamètre interne pour une composition contenant de préférence de 18 à 25 %, plus préférentiellement de 18 à 22%, plus préférentiellement encore de 18 à 21% de lanréotide, toujours inférieur à la SIF du produit commercial à 25% de lanréotide.

De préférence, la SIF de la composition pharmaceutique en tant que telle ou présente dans une seringue pré-remplie selon la présente demande va de 7 à 9 N.

Selon un mode de réalisation préféré, la composition pharmaceutique en tant que telle ou présente dans une seringue pré-remplie selon la présente demande présente une injectabilité définie par une SIF allant de 9 à 14 N lorsqu'elle est testée avec la méthode définie ci-dessus et une aiguille de 0,6 mm de diamètre interne pour une composition contenant de préférence de 18 à 25 %, plus préférentiellement de 18 à 22%, plus préférentiellement encore de 18 à 21% de lanréotide.

De préférence, la SIF de la composition pharmaceutique en tant que telle ou présente dans une seringue pré-remplie selon la présente demande va de 9 à 12 N.

Par comparaison, une composition commerciale de 25% en lanréotide ne peut pas être extrudée manuellement d'un dispositif comprenant une seringue ayant un diamètre supérieur à 3,50 mm. Pour injecter une telle composition, il faudrait utiliser une seringue de 3,50 mm de diamètre, couplée à une aiguille de 20mm de longueur et de 1,00 mm de diamètre interne et appliquer une force supérieure à 14N, en injectant très lentement

La force d'injection mesurée en newtons avec un dispositif différent du dispositif commercial dans les mêmes conditions dans tous les cas, passe par exemple de 14,3N avec une composition à 24,6% représentative de la forme Somatuline^{®} commerciale à 5,5N pour une composition à 19% de lanréotide dans l'eau.

Sachant qu'il existe une force résiduelle de l'ordre de 2N liée aux frictions du dispositif lors de l'injection à vide, c'est une réduction de plus de 70% de la force nécessaire à l'injection de ces 2 compositions pourtant proches, ce qui démontre un seuil utile de propriété exploitable selon la demande. De façon inattendue, une diminution relativement faible de la concentration entraine une diminution très importante de la force d'injection

En termes de force d'implantation, la diminution du diamètre de l'aiguille entraine une réduction qui peut faire une différence décisive entre une aiguille facilement implantable y-compris par le patient lui-même et un geste d'implantation compliqué qui nécessite l'expérience, la technique et la pratique d'un spécialiste.

Les forces d'implantation des aiguilles sont proportionnelles aux calibres ou gauges desdites aiguilles. Une différence apparemment faible de diamètre comme entre 1,00 mm et 1,20 mm correspond à une surface d'impact ou une section 1,5 fois plus grandes.

L'importance de réduire au maximum possible le diamètre de l'aiguille d'injection pour éviter la douleur et faciliter l'injection est illustrée dans l'article « Fracture mechanics model of needle cutting tissue » de Barnett et al. (2015), dans lequel les auteurs démontrent qu'il existe un seuil de force de pénétration ou d'insertion des aiguilles à travers la peau :
- Pour des aiguilles de diamètre externe allant de 1,60 mm (16G) à 1,20 mm (18G), les forces sont comparables et varient entre 2,5 et 3 N, ce qui pourrait être jugé élevé par l'homme de l'art ;
- Pour des aiguilles de diamètre externe allant de 0,80 mm (21G) à 0,50 mm (25G), les forces sont diminuées par deux, et varient entre 1 et 1,5 N.

De fait, ces différences apparemment faibles de diamètre correspondent à une perte de charge et donc à une augmentation de la force d'injection qui pour un même produit peut rendre impossible une administration manuelle.

Une autre limite des aiguilles utilisées dans les présentations commerciales actuelles telles que Somatuline^{®} est aussi liée à la nécessité d'utiliser des canules à parois fines de par exemple 0,10 mm, pour ne pas augmenter le diamètre externe. Cela réduit les possibilités d'avoir un biseau long et travaillé pour être tranchant et ainsi augmente les risques de déformation et carottage lors de l'implantation. Tout cela peut obliger à avoir recours à des techniques inhabituelles et compliquées, telles que celles consistant à tirer sur la peau avant implentation de l'aiguille. Tous ces effets sont susceptibles de compliquer et de rendre plus douloureuse l'introduction de l'aiguille dans les tissus. Un des avantages de la demande est donc aussi de pouvoir revenir à des canules de parois plus épaisses, par exemple comprise de 0,10 à 0,20 mm, de préférence de 0,15 mm.

En effet, le biseau d'une aiguille détermine comment se fera l'incision qui permettra la pénétration de la canule pour injecter le produit. Sur une aiguille de plus de 1,00 mm de diamètre externe, il est plus intéressant d'avoir des parois épaisses pour avoir plus de liberté sur le design de son biseau: en effet, avec des parois fines il est difficile de façonner librement la pointe de l'aiguille, ce qui peut rendre l'incision compliquée, et donc douloureuse pour le patient.

En utilisant des aiguilles standards (1,00 mm et moins de diamètre externe) selon la demande, il est possible de modifier le biseau à façon, et donc d'améliorer le confort de l'utilisateur pour une aiguille de diamètre externe qui reste inférieur à celui des aiguilles des compositions commerciales actuelles.

Enfin, une aiguille de 1,00 mm et moins de diamètre externe implique moins de force de pénétration et peut donc comporter un biseau réalisé sur une épaisseur de paroi de seulement 0,10 mm, réduisant ainsi son diamètre externe par rapport au diamètre interne.

A contrario, une aiguille de 1,20 mm et plus de diamètre externe implique plus de force de pénétration et nécessiterait plutôt un biseau réalisé sur une épaisseur de 0,15 mm, augmentant encore son diamètre externe par rapport au diamètre interne.

Une composition jusqu'à 21% ou 22% ou encore 25% de lanréotide peut être chargée classiquement avant mise en place du piston, par exemple de butyl dans une seringue BD Hypak^{™} Long de 1 mL bouchée par un bouchon luer-lock, ou d'une seringue en plastiques de 0,5mL en COP de type Clearject^{®} (Gerresheimer). Il est possible de pratiquer les injections en fixant sur l'extrémité luer-lock une aiguille standard de 21G de 25 mm ou de 16 mm pour obtenir les mêmes profils à différentes conditions d'injections et de profondeurs, ceci aussi grâce aux conditions de dépôts.

Selon un autre objet de la demande, la composition pharmaceutique en tant que telle ou présente dans une seringue pré-remplie comprend en outre un alcool, de préférence de l'éthanol. L'homme de métier aurait pu penser que l'ajout de solvants organiques même en concentrations faibles d'environ 5 à 10% en poids dans l'eau aurait conduit à des problèmes de stabilité.

Les inventeurs ont découvert qu'il est possible dans des présentations selon la demande d'utiliser ce type de mélange avec éthanol à une concentration d'au moins 5% et jusqu'à 25% dans l'eau qui permet d'obtenir des compositions de lanréotide stables et homogènes.

Avec des pourcentages faibles d'alcool qui ne posent pas de problème de douleur à l'injection ni de tolérance locale, les inventeurs ont découvert de façon inattendue que ces compositions sont plus visqueuses que celles obtenues avec l'eau seule à la même concentration de lanréotide, expliquant peut-être la stabilité du principe actif dans ces conditions.

Les compositions pharmaceutiques en tant que telles ou présentes dans une seringue pré-remplie selon la présente demande comprenant de 3 à 23% et préférentiellement de 3 à 10% d'éthanol permettent par contre d'utiliser et de valider plus facilement des procédés pour des préparations aseptiques prête à l'emploi, sans avoir recours à la gamma-irradiation en stérilisation terminale. Elles permettent aussi d'envisager des présentations solides de lanréotide stable à température ambiante avec des préparations extemporanées simples et rapides du produit à injecter.

Selon un autre mode de réalisation préféré, la composition pharmaceutique en tant que telle ou présente dans une seringue pré-remplie selon la présente demande, comprend de l'éthanol à une concentration de 3 à 23% en poids par rapport au poids total de la composition, de préférence de 3 à 10% en poids par rapport au poids total de la composition.

Selon un autre mode de réalisation préféré, la composition pharmaceutique en tant que telle ou présente dans une seringue pré-remplie selon la présente demande contenant du lanréotide à 20% en poids, de l'éthanol à une concentration de 5 à 25% en poids dans l'eau présente une injectabilité définie par une SIF allant de 7 à 9 N lorsqu'elle est testée avec la méthode définie ci-dessus

De préférence, le SIF de la composition pharmaceutique va de 7 à 8 N.

Par exemple, le test de force d'injection réalisé avec des compositions pharmaceutiques contenant du lanréotide à 20% en poids, et 5% ou 10% d'éthanol dans l'eau, montre des forces de 8,3 N et de 7,3 N respectivement.

Ces forces sont près de 50% inférieures à celle d'un mélange représentatif du produit commercial à 25%. Si l'on considère qu'une force d'injection à la main autour de 15 N représente un maximum acceptable dans les conditions du test, toute réduction de force proposée par une présentation selon la demande qui s'éloigne suffisamment de ce maximum constitue une amélioration importante.

Avec ces nouvelles compositions à base de mélange eau-éthanol, il est aussi possible de préparer des concentrations de lanréotide plus élevées, par exemple proches de 25%, comparables aux produits commerciaux, et d'utiliser une proportion d'éthanol plus grande mais toujours en faible quantité pour être injectable sans problème par exemple inférieur ou égal à 25% dans l'eau. On obtient alors une composition beaucoup plus fluide que les produits actuels (voir dans l'Exemple 9). Selon le test de force d'injection, une telle composition peut être injectée par exemple avec une aiguille de 0,80 mm de diamètre interne avec une force de 7,3 N, soit 70% inférieure à celle d'un mélange représentatif du produit commercial à 25% de lanréotide.

La demande a donc aussi pour objet une composition pharmaceutique en tant que telle ou présente dans une seringue pré-remplie selon la présente demande contenant du lanréotide à une concentration d'environ 18% à 25% et de l'éthanol à une concentration de 3 à 23% en poids ces pourcentages étant exprimés par rapport au poids total de la composition.

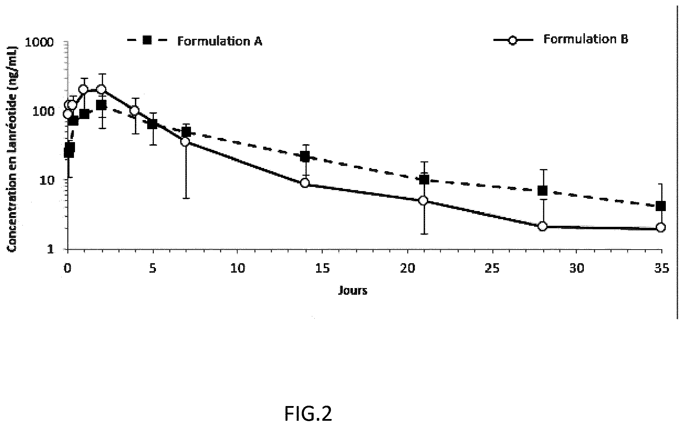

Les résultats obtenus sur l'animal et présentés dans les exemples, indiquent que les compositions eau-éthanol de la présente demande, comparées aux formes commerciales, permettent d'envisager de nouveaux profils de relargage du lanréotide, et permettent donc aussi par exemple de réduire le burst ou Cmax de 1,5 à 2 fois par rapport au produit commercial, ce qui permet aussi de multiplier par au moins 2 les niveaux circulants de lanréotide 2 à 4 semaines après l'injection. (Voir Figures 2-4)

De préférence les compositions, kits ou seringues pré-remplies selon la demande qui ne comprennent pas d'éthanol comprennent de 18 à 25%, plus préférentiellement de 18 à 22%, plus préférentiellement encore de 18 à 21% en poids de lanréotide par rapport au poids total de la composition, et celles comprenant de l'éthanol comprennent de 18 à 25% de lanréotide en poids de lanréotide par rapport au poids total de la composition.

Selon un autre mode de réalisation les compositions de la demande comprennent au moins un acide aminé, de préférence l'arginine, présent à une concentration allant de 0,1 à 13% en poids par rapport au poids total de la composition.

Nous avons aussi démontré qu'en fonction des proportions et conditions de mélange (type de milieu, température et temps) du lanréotide avec au moins un acide aminé, il est possible d'obtenir un profil de libération du lanréotide mieux contrôlé que celui des produits actuels tels que Somatuline^{®}, et d'une durée d'action d'une semaine à plusieurs mois.

Cette approche avec ajout d'un acide aminé peut conduire à différentes compositions nouvelles. On peut par exemple ajouter de l'arginine à des compositions comprenant différentes concentrations de lanréotide par exemple à une concentration égale ou inférieure à 21% ou 22% ou encore 25% dans l'eau.

La quantité en poids d'arginine pourra varier entre 0,1 et 13% par rapport au poids total de la composition.

Selon un autre mode de réalisation préféré, la composition pharmaceutique en tant que telle ou présente dans une seringue pré-remplie selon la présente demande est destinée à une libération prolongée du lanréotide pendant au moins 1 mois et comprend ou consiste essentiellement en :
- 18%, 18,5%, 19%, 19,5%, 20%, 20,5%,21%, 21,5%, 22%, 22,5%, 23%, 23,5%, 24%, 24,5% ou 25% en poids de lanréotide ;
- de 0 à 23% en poids d'éthanol, de préférence de 3% à 10% en poids ;
- de 0 à 13% en poids d'arginine, de préférence 1% à 13% en poids, encore plus de préférence 2% à 13% en poids.

Il est également possible d'adapter le profil de relargage en fonction des besoins, à partir d'une même composition, en choisissant la voie d'administration sous-cutanée (SC) ou intramusculaire (IM). L'ensemble de ces particularités avantageuses et nouvelles constituent aussi d'autres objets de la demande.

Toutes ces formes diluées associées à des aiguilles plus fines ne donnent pas des dépôts en volume contrôlés pour la gélification in situ mais conduisent à de plus fortes diffusions ou distributions au point d'injection ou encore à des dépôts de forme plus aplatie, suite à une précipitation in-situ du lanréotide.

On aurait pu s'attendre dans ces conditions à obtenir des relargages rapides ou non-contrôlés par exemple par solubilisation du lanréotide, mais de façon inattendue, cette augmentation de la surface d'échange entraîne la formation d'un précipité de plus faible volume qui contrôle différemment le relargage et ceci d'une façon utile et reproductible pour solutionner les différents problèmes décrits précédemment avec des injections plus classiques.

Une composition pharmaceutique en tant que telle ou présente dans une seringue pré-remplie selon la présente demande est administrée par voie parentérale.

Selon un mode de réalisation préféré, la composition pharmaceutique en tant que telle ou présente dans une seringue pré-remplie selon la présente demande est administrée par injection sous-cutanée, intramusculaire ou sous-cutanée profonde, et plus préférablement par injection sous-cutanée, ou par injection intramusculaire.

Les inventeurs ont découvert comme composition nouvelle, entre-autres la possibilité d'une composition plus diluée à 21% ou 22% ou encore 25% de lanréotide au maximum qui, injectée en sous-cutané, va permettre d'obtenir des niveaux plus plats avec un niveau final ou Cmin plus haut que les formes actuelles, que ce soit en injections toutes les 2 ou toutes les 4 semaines. Ce type de profil est utile par exemple en acromégalie et est aussi susceptible de résoudre des problèmes de moindre efficacité vers la fin des intervalles entre chaque injection. La même forme injectée en intramusculaire va permettre d'obtenir des niveaux et des Cmin plus hauts que les formes actuelles, avec un profil qui permet d'adapter les besoins à chaque patient en fonction d'un temps entre les injections spécifique à chaque cas, même pour une durée inférieure à 2 semaines pour les besoins de plus hautes doses comme cela est utile par exemple en NET (Neuro endocrine tumors, tumeurs neuroendocrines). Ces nouvelles présentations sont aussi très avantageuses pour les applications locorégionales du lanréotide comme en ophtalmologie, en diabétologie ou en dermatologie.

Toutes ces présentations nouvelles sont plus faciles à injecter et certainement moins douloureuses que les produits actuels autant par l'injection avec une aiguille plus fine que par le dépôt moins compact ou résistant, donc plus plat. Elles sont donc compatibles même avec des injections plus rapprochées. Comme indiqué précédemment, on sait que la force d'injection peut alors être par exemple plus de trois fois plus basse qu'avec les produits commerciaux ce qui favorise, voire même rend possible l'administration par le patient lui-même ou par son entourage avec un dispositif manuel type seringue standard.

Une composition pharmaceutique en tant que telle ou présente dans une seringue pré-remplie selon la présente demande est administrée par voie parentérale.

Selon un mode de réalisation préféré, la composition pharmaceutique en tant que telle ou présente dans une seringue pré-remplie selon la présente demande est administrée par injection sous-cutanée, intramusculaire ou sous-cutanée profonde, et plus préférablement par injection sous-cutanée ou intramusculaire. Pour la voie sous-cutanéequi reste préférée, le volume d'injection n'est de préférence pas supérieur à 2 ou 1,9 ou 1,8 ou 1,7 ou 1,6 ou 1,5 ou 1,4 ou 1,3 ou 1,2 ou 1,1 ou 1 mL d'une composition de lanréotide à une concentration allant de 18 à 25 %, plus préférentiellement de 18 à 22%, plus préférentiellement encore de 18 à 21% en poids de lanréotide par rapport au poids total de la composition.

Selon un mode de réalisation préféré, la libération prolongée de lanréotide est d'au moins 2 mois chez l'homme.

Selon un mode de réalisation préféré, la libération prolongée de lanréotide est d'au moins 3 mois chez l'homme.

La composition pharmaceutique en tant que telle ou présente dans une seringue pré-remplie selon la présente demande permet une libération prolongée pendant au moins 7 jours, 7 à 14 jours, 14 à 28 jours, 2 mois soit 60 jours ou plus de 2 mois soit 61, 62, 63, 64, 65, 66, 67, 68, 69, 70, 71, 72, 73, 74, 75, 76, 77, 78, 79, 80, 81, 82, 83, 84, 85, 86, 87, 88, 89 ou 90 jours.

Selon un mode de réalisation préféré, la composition pharmaceutique en tant que telle ou présente dans une seringue pré-remplie selon la présente demande permet une libération prolongée pendant au moins 60 jours.

Selon un autre mode de réalisation préféré, la composition pharmaceutique en tant que telle ou présente dans une seringue pré-remplie selon la présente demande permet une libération prolongée pendant au moins 7 jours.

Selon un autre mode de réalisation préféré, la composition pharmaceutique en tant que telle ou présente dans une seringue pré-remplie selon la présente demande permet une libération prolongée pendant au moins 90 jours.

Selon un mode de réalisation plus préféré, la composition pharmaceutique en tant que telle ou présente dans une seringue pré-remplie selon la présente demande permet une libération prolongée pendant au moins 14 jours.

Selon un autre mode de réalisation davantage préféré, la composition pharmaceutique en tant que telle ou présente dans une seringue pré-remplie selon la présente demande permet une libération prolongée pendant au moins 28 jours.

Comme indiqué précédemment, le lanréotide est un analogue commercialisé de la somatostatine. Il est indiqué dans le traitement de l'acromégalie due aux tumeurs hypophysaires et non hypophysaires sécrétant des hormones de croissance, à la prise en charge des symptômes et au traitement antiprolifératif des tumeurs neuroendocrines, en particulier des tumeurs carcinoïdes et des VIPomes, et au traitement de l'adénome thyrotrophique.

Une composition pharmaceutique en tant que telle ou présente dans une seringue pré-remplie selon la présente demande peut donc être utile dans le traitement de l'acromégalie due à des tumeurs hypophysaires et non hypophysaires sécrétant une hormone de croissance, à la gestion des symptômes provoqués par des tumeurs neuroendocrines ainsi que son contrôle, en particulier des tumeurs carcinoïdes et des VIPomes ou pour le traitement de l'adénome thyréotrope.

Selon un mode de réalisation préféré, une composition pharmaceutique en tant que telle ou présente dans une seringue pré-remplie selon la présente demande est utile dans le traitement de l'acromégalie ou des tumeurs neuroendocrines, par exemple, les tumeurs neuroendocrines gastroentéropancréatiques fonctionnelles et non fonctionnelles.

Selon les modalités de la demande, un patient souffrant d'acromégalie ou de NETs peut être traité par administration d'une quantité thérapeutiquement active de la composition pharmaceutique décrite dans l'un quelconque des modes de réalisation décrits ci-dessous.

Selon un mode de réalisation, la composition pharmaceutique en tant que telle ou présente dans une seringue pré-remplie selon la présente demande est administrée ou injectée, ou préparée pour administration ou injection chez un patient en ayant besoin, pas plus fréquemment que toutes les 2 semaines ou de préférence tous les 4 ou 8 ou 12 ou 16 semaines. De préférence, il est administré ou injecté toutes les 4 semaines.

Selon un autre mode de réalisation préféré, la composition pharmaceutique en tant que telle ou présente dans une seringue pré-remplie selon la présente demande est administrée ou préparée pour une administration en une seule injection.

De préférence, le procédé comprend l'administration de la composition pharmaceutique en tant que telle ou présente dans une seringue pré-remplie selon la présente demande au moins une fois toutes les 2 semaines ou 4 semaines ou 8 semaines ou 12 semaines ou 14 semaines ou 16 semaines. Plus préférablement, le procédé comprend l'administration de la composition pharmaceutique en tant que telle ou présente dans une seringue pré-remplie selon la présente demande au moins une fois toutes les 12 semaines.

Selon un autre mode de réalisation, la composition pharmaceutique en tant que telle ou présente dans une seringue pré-remplie selon la présente demande est administrée par injection unique. De préférence, la composition pharmaceutique en tant que telle ou présente dans une seringue pré-remplie selon la présente demande est injectée par voie intramusculaire, sous-cutanée ou sous-cutanée profonde.

Selon un autre mode de réalisation, la composition pharmaceutique en tant que telle ou présente dans une seringue pré-remplie selon la présente demande est administrée à une dose de 60, 90, 120 ou 240 ou 360 mg de lanréotide. De préférence, la composition pharmaceutique en tant que telle ou présente dans une seringue pré-remplie selon la présente demande est administrée à une dose de 120, 240 ou 360 mg de lanréotide. Les avantages de cette demande qui permettent des injections plus faciles avec des aiguilles plus fines et des dépôts in-situ plus petits moins perceptibles donc plus confortables permettent de préparer ces doses plus importantes pour une seule injection, comme par exemple le double ou plus des doses des produits commerciaux, doses qui correspondent à un besoin de traitement par exemple pour les tumeurs neuroendocrines.

Selon les modalités de la demande un patient souffrant d'une tumeur neuroendocrine peut être traité par l'administration d'une quantité thérapeutiquement active de la composition pharmaceutique décrite dans l'un quelconque des modes de réalisation ci-dessus. De préférence, la composition pharmaceutique en tant que telle ou présente dans une seringue pré-remplie selon la présente demande est administrée pour le contrôle antiprolifératif des tumeurs neuroendocrines, ou pour éviter les symptômes provoqués par ces tumeurs neuroendocrines.

Selon un mode de réalisation, ces symptômes sont choisis parmi les bouffées congestives, la diarrhée et les crampes abdominales.

Selon les modalités de la demande un patient souffrant d'adénome thyrotrophique peut être traité par l'administration d'une quantité thérapeutiquement active de la composition pharmaceutique décrite dans l'un quelconque des modes de réalisation ci-dessus.

Selon un autre mode de réalisation, cette demande permettant la formation de dépôts plus petits et plus de précision dans les dosages car moins concentrés et administrés avec des aiguilles plus fines, et permet ainsi de préparer des doses plus petites et moins visqueuses par exemple pour les applications du lanreotide en ophtalmologie, par exemple de 20 mg ou moins ou de 0,1 mL ou moins, pour les patients souffrant de glaucome, d'oedème maculaire, d'inflammation, de rétinopathie diabétique ou de dégénérescence maculaire liée à l'age. Ces caractéristiques s'adaptent aussi aux applications du produit en dermatologie comme par exemple pour le traitement des kéloïdes.

Les avantages des seringues et formulations selon cette demande permettent aussi de réaliser ces injections très rapidement avec une faible course du piston pour ces faibles volumes.

Selon les modalités de la demande, ces formulations moins visqueuses avec une charge de lanreotide plus basse permettent aussi de réaliser plus efficacement des combo-compositions dans lesquels un ou plusieurs autres principes actifs sont ajoutés au lanreotide qui peut contrôler aussi leurs relargages combinés pour le même traitement. Par exemple en ophtalmologie un glucocorticoïde comme dexaméthasone ou un anticorps monoclonal ou le mélange de 2 ou plusieurs de ces principes actifs.

La composition pharmaceutique telle que décrite ci-dessus est préparée par un procédé comprenant les étapes suivantes :
- préparation du solvant aqueux comprenant de l'eau et optionnellement de l'éthanol et/ou un acide aminé, par exemple l'arginine, dans une seringue
- introduction du lanréotide dans un récipient approprié, par exemple dans une seconde seringue ou dans un récipient en forme de seringue,
- connexion des 2 seringues ou des récipients de type seringue avec un connecteur à 2 voies, et hydratation du lanréotide avec le solvant aqueux et homogénéisation de la composition au moyen d'un procédé de mélange à une température comprise entre 5 et 70 °C, de préférence à température ambiante.

De plus, l'ensemble du processus de préparation peut être contrôlé en ce qui concerne les paramètres de traitement critiques tels que la température, la pression, le nombre de cycles et le rapport seringue-diamètre de valve, avec un équipement connu de l'homme de l'art.

Selon un autre mode de réalisation de la demande, l'ingrédient actif peut être préalablement traité seul ou avec l'arginine dans un milieu aqueux dans des proportions et du temps qui varient selon l'obligation de contrôle de relargage. Le lanréotide peut par exemple être traité par lyophilisation, séchage, broyage, granulation, compactage, tamisage, avant la préparation de la composition pharmaceutique en tant que telle ou présente dans une seringue pré-remplie selon la présente demande.

Toute technique de stérilisation telle que l'irradiation gamma, l'irradiation par faisceau d'électrons, la vapeur ou la filtration stérile pour une partie du procédé peut être utilisée pour obtenir une composition pharmaceutique stérile.

Selon un autre mode de réalisation de la demande, la préparation de la composition pharmaceutique en tant que telle ou présente dans une seringue pré-remplie selon la présente demande est conduite dans des conditions aseptiques.

Une composition pharmaceutique selon la présente demande peut être disponible sous forme de présentation pré-remplie et prête à l'emploi. Elle peut également être disponible sous forme de présentation prête à reconstituer où un produit lyophilisé contenant l'ingrédient actif peut être reconstitué extemporanément avec le solvant aqueux, et d'autres additifs si nécessaire comme solvant pour la reconstitution.

En tant qu'autre mode de réalisation, la composition pharmaceutique peut être conditionnée dans des seringues pré-remplies.

La demande concerne également un kit comprenant une composition pharmaceutique à libération prolongée et/ou contrôlée pour administration parentérale, ladite composition comprenant:
a) un solvant aqueux constitué d'eau et éventuellement d'un solvant organique miscible à l'eau de préférence un alcool, encore plus préférentiellement l'éthanol
b) du lanréotide ou un sel de lanréotide, de préférence l'acétate de lanréotide à une concentration allant de 11 à 25% en poids, de préférence de 18 à 25 %, plus préférentiellement de 18 à 22%, plus préférentiellement encore de 18 à 21% en poids de lanréotide par rapport au poids total de la composition,
c) éventuellement un ou plusieurs acides aminés, de préférence l'arginine,
d) éventuellement un excipient monomérique pharmaceutiquement acceptable,
et une seringue dont l'aiguille possède un diamètre externe égal ou inférieur à 1,00 mm, un diamètre interne égal ou inférieur à 0,80 mm et une longueur comprise de préférence entre 10 et 40mm.

Dans les conditions nouvelles de cette demande, des dépôts sont obtenus par précipitation du lanréotide après interaction avec les tissus et fluides corporels du patient au site d'injection. Ce type de dépôt plus petit et plus plat est mieux toléré.

De façon inattendue, le lanréotide utilisé dans des formes plus diluées que les formes semi-solides ou pâteuses des compositions commerciales actuelles peut être associé dans une présentation à un dispositif d'injection avec une aiguille plus fine que les aiguilles actuelles d'au moins 1,20 mm de diamètre externe. L'injection avec cette nouvelle présentation conduit à un dépôt différent qui donne des formes contrôlées avec des propriétés nouvelles et avantageuses, utiles à tous les besoins thérapeutiques du produit et capables de solutionner les limites ou problèmes posés par les formes contrôlées actuelles.

Selon un autre mode de réalisation préféré, la demande concerne une composition pharmaceutique comprenant :
a) un solvant aqueux constitué d'eau et éventuellement d'un solvant organique miscible à l'eau de préférence un alcool, encore plus préférentiellement l'éthanol,
b) du lanréotide ou un sel de lanréotide, de préférence l'acétate de lanréotide à une concentration allant de 11 à 25% en poids, de préférence de 18 à 25 %, plus préférentiellement de 18 à 22%, plus préférentiellement encore de 18 à 21% de lanréotide en poids par rapport au poids total de la composition,
c) éventuellement un ou plusieurs acides aminés, de préférence l'arginine,
d) éventuellement un excipient monomérique pharmaceutiquement acceptable,
ladite composition étant caractérisée en ce que, lorsqu'elle est administrée par injection sous cutanée, normale ou profonde, ou intramusculaire chez un patient à l'aide d'une seringue ayant un diamètre interne de l'ordre de 3,50 mm, plus préférentiellement supérieur à 4,00 mm jusqu'à 7, 00 mm de diamètre et est équipée d'une aiguille ayant un diamètre externe égal ou inférieur à 1,00 mm et un diamètre interne égal ou inférieur à 0,80 mm et une longueur comprise de préférence entre 10 et 40 mm de préférence une longueur de 20 à 30 mm, libère le lanréotide ou le sel de lanréotide sur une durée d'au moins 7 jours, de préférence d'au moins 28 jours.

De essais sur animaux ont été effectués afin de démontrer que les dépôts obtenus, même si différents, maintenaient l'effet de relargage contrôlé ou retard de lanréotide.

En fonction des objectifs, il est possible de réaliser de nombreux produits nouveaux différents avec les compositions de lanréotide et les kits ou seringues pré-remplies possibles selon la demande.

Sans que cela soit limitatif, on peut à titre d'exemples mentionner les différents types de possibilités suivants :
- Un profil de relargage du lanréotide similaire à celui des formes commerciales actuelles telles que Somatuline^{®} sur 4 semaines avec une composition contenant 20% de lanréotide dans l'eau et des doses jusqu'à 120 mg dans une seringue de 3,50 mm de diamètre équipée d'une aiguille de 0,80 mm de diamètre externe et de 20 mm de longueur pour une administration sous cutanée.
- Un profil de relargage du lanréotide plus élevé que les formes actuelles telles que Somatuline^{®} sur 4 semaines avec une composition contenant 20% de lanréotide dans l'eau et des doses de 240mg ou plus dans une seringue de 3,50 mm de diamètre interne équipée d'une aiguille de 1,00 mm de diamètre externe et de 20 mm de longueur pour une administration sous-cutanée.
- Un profil de relargage du lanréotide plus élevé que les formes actuelles telles que Somatuline^{®} sur 2 semaines avec une composition contenant 20% de lanréotide dans l'eau et des doses jusqu'à 120mg dans une seringue de 3,50 mm de diamètre équipée d'une aiguille de 0,80 mm de diamètre externe et de 40 mm de longueur pour administration intramusculaire.
- Un profil de relargage du lanréotide similaire à celui des formes actuelles telles que Somatuline^{®} sur 4 semaines avec une composition contenant 20% de lanréotide dans l'eau et des doses jusqu'à 100 mg dans une seringue de 3,00 mm de diamètre équipée d'une aiguille de 0,60 mm de diamètre externe et de 20 mm de longueur pour administration sous cutanée.
- Un profil de relargage similaire à celui des formes actuelles telles que Somatuline^{®} sur 4 semaines ou plus avec une composition contenant 20% dans l'eau et des doses de 20mg ou moins dans une seringue de 3,00 mm de diamètre ou moins équipée d'une aiguille de 0,60 mm de diamètre externe ou moins.
- Un profil de relargage du lanréotide plus élevé que les formes actuelles telles que Somatuline^{®} sur 2 semaines avec une composition contenant 20% de lanréotide dans l'eau et des doses jusqu'à 100 mg dans une seringue de 3,00 mm de diamètre équipée d'une aiguille de 0,70 mm de diamètre externe et de 30 mm de longueur pour administration intramusculaire.
- Un profil de relargage du lanréotide similaire à celui des formes actuelles telles que Somatuline^{®} ou plus élevé que les formes actuelles sur 4 ou 2 semaines avec compositions à 20% dans l'eau jusqu'à plus de 120 mg dans une seringue standard pré-remplie classiquement sur laquelle on fixe une aiguille luer-lock sous cutanée courte ou intramusculaire longue de 21 ou 20G.
- Un profil de relargage du lanréotide similaire à celui des formes actuelles telles que Somatuline^{®} ou plus élevé que les formes actuelles sur 4 ou 2 semaines avec une composition contenant 20% de lanréotide dans un mélange 5:95 alcool/eau jusqu'à plus de 120 mg en seringue standard fabriquée aseptiquement préparée extemporanément stable à température ambiante pour administrations sous cutanée ou intramusculaire.
- Un profil de relargage du lanréotide plus plat que les formes actuelles telles que Somatuline^{®} sur 4 ou 8 semaines avec une composition contenant 25% de lanréotide dans un mélange 25:75 alcool/eau jusqu'à plus de 120 mg en seringue standard fabriquée aseptiquement préparée extemporanément stable à température ambiante pour administration sous cutanée ou intramusculaire.
- Un profil de relargage du lanréotide plus plat que les formes actuelles telles que Somatuline^{®} sur 2, 4, 8, ou 12 semaines avec une composition contenant 20% de lanréotide et 12% d'arginine dans l'eau jusqu'à plus de 120 mg en seringue standard pré-remplie classiquement sur laquelle on fixe une aiguille luer-lock sous cutanée courte ou intramusculaire longue.

### DESCRIPTION DES FIGURES

La Figure 1 représente des extrudats comparables d'acétate de lanréotide obtenus après injection dans l'air d'une composition de lanréotide à 19% et 25% en poids contenus dans une seringue de 3,50 mm de diamètre équipée d'une aiguille de 1,00 mm de diamètre interne.
La Figure 2 représente l'évolution de la concentration plasmatique en lanréotide en fonction du temps pour les deux compositions A et B préparées selon l'Exemple 13
La Figure 3 représente l'évolution de la concentration plasmatique en lanréotide en fonction du temps pour la composition préparée selon l'Exemple 14
La Figure 4 représente l'évolution de la concentration plasmatique en lanréotide en fonction du temps pour la-composition préparée selon l'Exemple 15
La Figure 5 représente l'évolution de la concentration plasmatique en lanréotide en fonction du temps pour la composition préparée selon l'Exemple 17

### DESCRIPTION DÉTAILLÉE DE L'INVENTION

Il est possible de décrire l'invention avec plus de détails en précisant quelques exemples de réalisations selon cette invention.

### EXEMPLE 1

### Préparation de compositions entre 18 et 21% ou 22% ou encore 25% de lanréotide sous forme de lanréotide acétate dans deux types de solvant, l'eau et un mélange eau/alcool éthylique 95:5%

Les préparations sont réalisées par une technique de va-et-vient entre 2 réservoirs de type seringue fermés d'un côté par un piston et ouverts de l'autre par une partie connectée à une vanne permettant optionnellement de conditionner la poudre de lanréotide acétate sous vide, d'hydrater cette poudre avec lesdits solvants et de mélanger le produit par va-et-vient en régulant l'ouverture de la connexion par la vanne, ceci en mesurant optionnellement les forces exercées pour contrôler le type de mélange à réaliser.

En comparant les processus de réalisation de ces mélanges à ceux correspondant à la préparation d'une composition à 25% de lanréotide comparable au produit commercial, on constate qu'il est beaucoup plus facile et plus rapide de réaliser ces préparations entre 18 et 22% et que cela nécessite moins de force de mélange. Il est aussi alors possible d'extrapoler ce type de mélange à des volumes beaucoup plus grands, ou encore de réaliser ce mélange par d'autres techniques que celles spécifiquement adaptés aux mélanges semi-solides ou pâteux.

Le mélange est simplifié de telle manière qu'il est également possible selon des protocoles connus de l'homme de l'art de remplir des seringues comme cela se fait avec des formes liquides, ou de conditionner la dose requise dans chaque seringue sous forme de poudre ou de lyophilisé pour pouvoir l'hydrater ensuite extemporanément et la mélanger manuellement au moment de l'utilisation.

Cette autre présentation de lanréotide selon l'invention permet par exemple d'envisager des compositions à libération prolongée et/ou contrôlée stables à température ambiante ou encore des compositions à concentrations variables à partir d'une seule et même dose de lanréotide, pour obtenir des profils adaptés aux différents besoins thérapeutiques.

### EXEMPLE 2

### Composition à 19,0% de lanréotide dans l'eau

Lot préparé à partir de 1,88 g de lanréotide acétate et d'eau purifiée.

La préparation est réalisée par la technique de va-et-vient décrite dans l'Exemple 1, à l'aide de 2 seringues en plastiques connectées à un connecteur qui permet de réaliser le mélange après hydratation de la poudre de lanréotide acétate avec l'eau. La composition obtenue est repartie dans différents systèmes d'injection pour évaluer la force d'injection et d'autres paramètres comme le contenu en peptide et la pureté chromatographique.

### EXEMPLE 3

### Composition à 19,5% de lanréotide dans l'eau et alcool éthylique

Lot préparé à partir de 0,36 g de lanréotide acétate et d'un melange 95:5 d'eau purifiée et d'alcool éthylique. Le mélange eau-alcool à 95:5 se prépare en mélangeant 1,906g d'eau purifiée avec 0,103g d'alcool éthylique à 96%.

La préparation est réalisée par la technique de va-et-vient décrite dans l'Exemple 1, à l'aide de 2 seringues en plastique connectées à un connecteur qui permet de réaliser le mélange après hydratation de la poudre de lanréotide acétate avec le mélange eau/alcool éthylique. La composition obtenue est repartie dans différents systèmes d'injection pour évaluer la force d'injection et d'autres paramètres comme le contenu en peptide et la pureté chromatographique.

### EXEMPLE 4

### Description des mesures de force d'injection

Les mesures de force d'injection des compositions préparées comme décrit dans l'Exemple 1 sont réalisées à l'aide d'un dynamomètre (L1000R, Lloyd Instruments Ltd.) pourvu d'une cellule de capture de force calibrée (NLC 100N, Lloyd Instruments Ltd.) couplée à un système informatique pour l'acquisition et le traitement des données. Pour chaque mesure, le système d'injection (seringues + aiguilles) contenant la composition est placé sur un support en position verticale (aiguille vers le bas) et le dynamomètre est utilisé pour déplacer le piston dans la seringue pour simuler une injection. Les mesures sont réalisées à une vitesse de déplacement constante de 100 mm/min, la force détectée par la cellule est enregistrée en fonction du déplacement du piston. La composition déchargée lors de chaque test peut être récupérée dans une fiole jaugée pour compléter les analyses physico-chimiques comme par exemple le contenu en peptide des échantillons décrit dans l'Exemple 5.

### EXEMPLE 5

### Description du processus de détermination de contenu en peptide et de pureté chromatographique

Les compositions préparées comme décrit dans l'Exemple 1, 2 et 3 sont analysées par chromatographie liquide en mode RP-HPLC (de l'anglais reverse phase high-pressure liquid chromatography). Pour la détermination de la pureté chromatographique l'aire du pic principal correspondant à la lanréotide est comparée en % par rapport à l'aire de l'ensemble des pics détectés. Pour la détermination du contenu en peptide, l'aire du pic principal correspondant à la lanréotide est utilisée pour calculer la concentration de la composition injectée en tenant compte des réponses (aires) obtenues pour les injections de compositions de standards de référence de lanréotide. A partir de la concentration de la composition injectée, de la masse de l'échantillon analysé et du volume de dissolution, le contenu en peptide (% massique) de la composition peut être calculé.

Conditions et méthode RP-HPLC utilisés par les inventeurs :
- solvant pour la préparation des échantillons : 0,1% d'acide acétique dans de l'eau de qualité HPLC ou un mélange 85:15 d'acide acétique (0,1%) dans de l'eau de qualité HPLC et d'acétonitrile.
- colonne : C18 XBridge de 150 x 4.6 mm
- Température colonne: 35ºC
- Phase mobile A : 0,1% d'acide trifluoroacétique (TFA) dans de l'eau de qualité HPLC
- Phase mobile B : mélange 80:20 acétonitrile/eau de qualité HPLC à 0,1% TFA
- Débit: 0,9mL/min
- Elution : gradient de B dans A (15% à 53.7% en 21,5 min puis 70% à 22,5 min puis 85.0% à 23,5 min)
- Volume d'injection : entre 10 et 100µL
- Concentration des compositions : entre 80 et 800 µg/mL en lanréotide
- Détection : UV a 280 nm

### EXEMPLE 6

### Conditionnement des préparations dans des seringues fines type insuline commerciale et dans des seringues ou réservoirs classiques pré-remplissables

Les inventeurs se sont rendus compte que le remplissage des seringues avec les compositions préparées comme décrit dans l'Exemple 1 était plus simple à réaliser, plus rapide et moins problématique qu'avec un mélange équivalent au produit commercial à 25%. Les différences sont si importantes qu'il est ici aussi possible de remplir les réservoirs de type seringues de façon traditionnelle comme dans le cas d'injectables liquides et d'utiliser des réservoirs standards de plus grand diamètre, tels que les cartouches ou seringues pré-remplissables du commerce, en verre ou en plastique. Il est par exemple aussi possible de préparer des doses pré-remplies dans des seringues standard en verre, comme par exemple de type HyPak^{™}, HyLok^{™} ou dans des seringues en plastiques en COP de type Clearject^{®}.

### EXEMPLE 7

### Description de la répartition d'une composition à 19% dans des seringues « fines » de type insuline

Un lot préparé tel que décrit dans l'Exemple 2 à 19% de lanreotide dans l'eau est rempli dans des seringues fines en plastique de type insuline de 3,50 mm de diamètre interne. Le remplissage des seringues se réalise par l'avant en connectant la seringue fine à l'extrémité d'une des grandes syringes utilisées pour la préparation de la composition. La charge de la composition déplace le piston de la seringue fine vers l'arrière lors de son introduction.

Pour des doses injectées de 30 mg de lanréotide avec une aiguille rapportée par la suite de 1,00(OD)x0,80(ID)x20mm (L), 162mg de composition sont chargés dans chaque seringue en plastique. L'aiguille est ensuite couplée à la seringue et protégée par un bouchon standard de butyle.

### EXEMPLE 8

### Description de la répartition d'une composition à 19% dans des seringues pré-remplissables du commerce

Un lot préparé tel que dans l'Exemple 2 à 19% de lanreotide dans l'eau est rempli dans des syringes en verre de 1,0 mL. Le remplissage des syringes en verre se réalise par l'arrière comme dans le cas d'un remplissage industriel en utilisant une canule en plastique de 3,50 mm de diamètre interne avec un diamètre externe inférieur au diamètre interne des seringues en verres.

Pour des doses injectées de 30 mg de lanréotide avec une aiguille rapportée par la suite de 21G (16 ou 25 mm de longueur), environ 225 mg de composition sont chargés au fond de chaque seringue en verre. Un piston de bromobutyl est ensuite positionné pour minimiser au maximum le volume d'air emprisonné entre la composition et le piston.

### EXEMPLE 9

### Exemple de mesures de forces d'injection

Les essais de force d'injection sont réalisés selon des protocoles calibrés et validés, décrits dans l'Exemple 4.

La force d'injection maximale exprimée en newtons (N) est dérivée des données recueillies pendant la décharge.

Dans cet exemple, sont comparées des aiguilles représentatives du produit commercial Somatuline (1,20 mm de diamètre externe et 1,00 mm de diamètre interne) avec des aiguilles de diamètre inférieur, ainsi que les forces d'injection de compositions préparées selon les exemples 1, 2 et 3 à travers les mêmes aiguilles et différents systèmes d'injection (seringue + aiguille).

Les résultats obtenus sont rapportés dans la table suivante :

| %(m/m) lanréotide | Solvant (eau:alcool) | Système d'injection (en mm) | Force d'Injection (Newton) |
|---|---|---|---|
| 25,0 | 100:0 | Diamètre interne seringue : 3,5 | 14,3 |
| | | Aiguille (Dext/Dint/L) : 1,2/1,0/20 | |
| 19,0 | 100:0 | Diamètre interne seringue : 3,5 | 5,5 |
| | | Aiguille (Dext/Dint/L) : 1,2/1,0/20 | |
| 18,7 | 100:0 | Diamètre interne seringue : 3,5 | 8,8 |
| | | Aiguille (Dext/Dint/L) : 1,0/0,8/20 | |
| 18,8 | 100:0 | Diamètre interne seringue : 3,5 | 11,9 |
| | | Aiguille (Dext/Dint/L) : 0,8/0,6/20 | |
| 25 | 75:25 | Diamètre interne seringue : 3,5 | 7,3 |
| | | Aiguille (Dext/Dint/L) : 1,0/0,8/10 | |
| 19,9 | 90:10 | Diamètre interne seringue : 3,5 | 7,3 |
| | | Aiguille (Dext/Dint/L) : 1,2/1,0/20 | |
| 19,5 | 95:5 | Diamètre interne seringue : 3,5 | 8,3 |
| | | Aiguille (Dext/Dint/L): 1,2/1,0/20 | |

En utilisant des compositions plus diluées malgré les aiguilles choisies beaucoup plus fines et longues que celles des produits commerciaux pour injections en sous-cutané profond, on a constaté dans les conditions de la demande, des diminutions de forces d'injections par au moins 3 ou 4.

### EXEMPLE 10

### Apparence visuelle et comparaisons d'extrudats obtenus par injection à travers différentes aiguilles

Pour démontrer l'importance des gros diamètres de l'aiguille selon les approches connues avant l'insertion on peut vérifier qu'avec les aiguilles d'au moins 1 mm de lumière ou diamètre interne des produits commerciaux à 25% de lanréotide avec une des compositions plus diluées, on obtient après injection in-vitro un extruda ou cylindre de ce même diamètre. Avec les aiguilles plus fines combinées aux compositions plus diluées, les inventeurs ont réussi à obtenir après injection in-vitro un produit qui ne garde pas cette forme, démontrant une capacité de cette nouvelle présentation avec une aiguille plus fine et une composition plus diluée à ne pas dépendre du dépôt pour contrôler le relargage du lanréotide.

Des essais ont été menés pour évaluer l'apparence visuelle et la géométrie d'extrudats de 2 compositions de lanréotide préparées telles que dans l'Exemple 1, à travers différentes aiguilles.

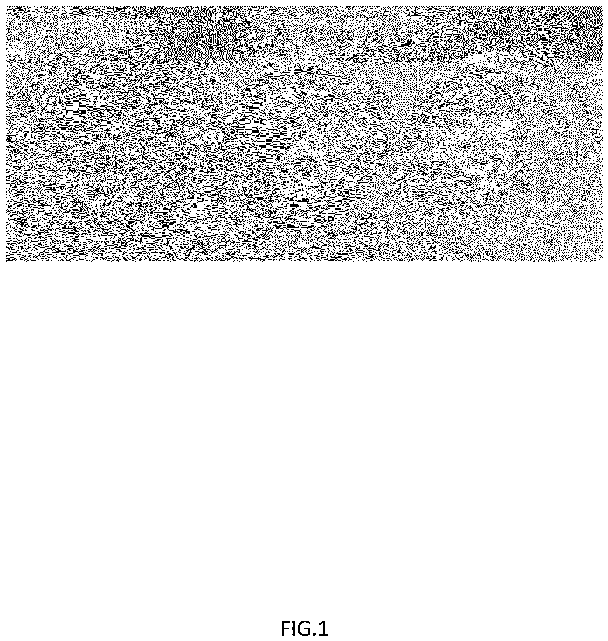

Les résultats sont visibles sur la Figure 1:
- À gauche un extrudat d'une composition à 25% de lanréotide a travers une aiguille de 1,00 mm de diamètre interne
- Au milieu un extrudat d'une composition à 19% de lanréotide a travers'une aiguille de 1,00 mm de diamètre interne
- A droite un extrudat d'une composition à 19% de lanréotide a travers une aiguille de 0,60 mm de diamètre interne

Les résultats obtenus avec une aiguille de diamètre interne de 1,00 mm donnent des extrudats similaires. Il n'a pas été possible de réaliser la même comparaison avec une aiguille de 0,60 mm : seule la composition à 19% de lanréotide peut être extrudée manuellement et l'extrudat est significativement différent.

### EXEMPLE 11

### Injection manuelle

Pour des doses de compositions préparées selon l'Exemple 2 à 19% de lanréotide pré-remplies dans des syringes standard en verre de 1,00 mL il a été possible de vérifier que l'injection manuelle peut être réalisé en utilisant une aiguille hypodermique standard de 21G de 25mm. Ces injections manuelles ont été réalisées en utilisant des appui-doigts standard et disponibles pour les seringues standard commerciales.

Les essais sont réalisés à partir d'aiguilles standards fixées extemporanément aux différentes seringues permettant d'envisager des injections sous-cutanées normales ou profondes en fonction de la longueur de l'aiguille variant de 10 à 20mm comme des injections intramusculaires avec des aiguilles longues de 25 à 40mm.

La combinaison des avantages d'implantation d'aiguille et d'administration de produit avec moins de force, de choix de la zone d'injection, de dépôt moins visqueux qui garde moins sa forme ou diffuse d'avantage et de réduction du volume par la précipitation du lanréotide sont autant de facteurs qui contribuent à pouvoir rendre ces traitements plus faciles, plus confortables, moins douloureux à administrer et mieux tolérés au site de dépôt en évitant les nodules sensibles.

### EXEMPLE 12

### Étude de stabilité des compositions à 20% de lanréotide dans l'eau et dans un mélange eau-éthanol.

Une composition a 25% de lanréotide préparée comme décrit dans l'Exemple 1 avec un mélange d'eau purifiée et d'alcool éthylique 75:25 a été testée en termes de stabilité à 5ºC et 25ºC/60% RH. Après 1 mois de de stockage dans ces conditions, la pureté du peptide déterminée par RP-HPLC est comparée à la pureté initiale. Les résultats sont reportés dans la table suivante.

| Pureté à t=0 | Pureté à t = 1 mois et stockage à 5ºC | Pureté à t = 1 mois et stockage à 25ºC/60%RH |
|---|---|---|
| 99,1%Ar | 99,1%Ar | 98,9%Ar |

La stabilité de différentes compositions a été étudiée selon des protocoles permettant de prévoir la faisabilité de présentations prêtes à l'emploi (ready-to-use) dans différentes conditions de fabrication, en aseptique ou avec stérilisation terminale. Il a été démontré que les compositions à 20% de lanréotide sont stables.

De façon inattendue si l'on considère d'autres données avec un pourcentage faible de solvant organique, il a été constaté que les compositions avec un mélange d'eau contenant 10% ou moins d'éthanol étaient également stables.

Il est donc possible avec ces 2 types de compositions à libération prolongée et/ou contrôlée moins visqueuses de proposer une présentation de produit injectable prête-à-l'emploi sans étapes de reconstitution du produit avant injection.

### EXEMPLE 13

### Profils pharmacocinétiques (PK) obtenus chez le rat

Afin de vérifier le fonctionnement et la faisabilité de nouveaux produits injectables à partir de ces nouvelles présentations, des études pharmacocinétiques sur des rats et sur des chiens ont été réalisées.

Les 2 compositions suivantes préparées selon l'Exemple 1 à parir d'acétate de lanréotide ont été testées sur le rat :
- Formulation A : 24,6% de lanréotide dans un mélange d'eau purifiée et d'alcool éthylique à 75:25
- Formulation B : 27,9% de lanréotide dans de l'eau purifiée.

Des doses aux alentours de 15 mg sont injectées en sous-cutané et des échantillons de sang prélevés tout au long de l'étude. Les différents échantillons de plasma dérivés des échantillons de sang sont analysés par LC-MS pour déterminer la concentration plasmatique en lanréotide.

Les résultats sont visibles sur la Figure 2, sur laquelle est tracée l'évolution de la concentration plasmatique en lanréotide en fonction du temps pour les deux compositions A et B.

Dans les deux cas, on observe un relargage prolongé du lanréotide. Cependant il est clairement visible que la composition A, bien que contenant un pourcentage plus faible de lanréotide donne une amélioration du profil de relargage : le burst initial ou Cmax est réduit, ce qui permet de multiplier par quasiment deux les niveaux de lanréotide à 4 semaines.

Il est possible de tester cette nouvelle présentation avec par exemple une aiguille de 1,00 mm de diamètre et une composition à 20% de lanréotide et la comparer avec les références connues d'une composition commerciale à 25% injectée avec une aiguille de 1,20 mm de diamètre.

Les résultats obtenus dans cet exemple laissent à penser qu'il est alors possible d'obtenir des profils de relargage comparables voire mieux contrôlés avec une nouvelle présentation selon la demande grâce à l'arrangement du dépôt dans son mode de précipitation. Il est alors possible d'estimer que ce type de présentation nouvelle avec les aiguilles appropriées peut permettre par exemple dans le cas de l'acromégalie des administrations en sous-cutanée tous les 28 jours avec un profil de relargage présentant moins de burst et un niveau plus haut en fin d'intervalle ; ou encore par exemple des administrations en intramusculaire tous les 14 jours avec un profil de relargage en tous points plus élevé suivi d'une baisse rapide des niveaux de lanréotide pour traiter les tumeurs neuro-endocrines.

Afin de vérifier le fonctionnement des nouvelles formes contrôlées, il est aussi possible de tester cette nouvelle présentation dans des conditions extrêmes de viscosité nettement plus basses, par exemple à partir d'une préparation à 20% de lanréotide dans un mélange eau-alcool à 95:5%. Dans ce cas, il est possible de montrer que l'on garde le contrôle du relargage vers ces limites et qu'il est aussi possible d'avoir ainsi des profils nouveaux plus avantageux, comme par exemple la possibilité d'obtenir des niveaux de relargages plus élevées, mieux adaptés et nécessaires pour certains traitements comme dans le cas des NETs.

### EXEMPLE 14

### Exemple de profil pharmacocinétique (PK) obtenue sur le chien

De manière générale, des présentations selon la demande sont susceptibles de donner des profils avec des doses administrées plus élevées sur les chiens que sur les rats, ainsi plus faciles à comparer aux doses utilisées chez l'homme en clinique.

Un lot préparé selon l'Exemple 1 à partir de lanréotide acétate dans l'eau purifiée est testé chez le chien et à une concentration de 20,2% de lanréotide. Des doses de 60mg (lanréotide) sont injectées en intramusculaire et des échantillons de sang sont prélevés tout au long de l'étude.

Les doses sont conditionnées dans des seringues de 3,0 mm de diamètre couplées à des aiguilles de 0,8x0,6x19mm. L'administration intramusculaire avec ces aiguilles de faible diamètre est réalisée sans problème.

Les différents échantillons de plasma dérivés des échantillons de sang sont analysés par LC-MS pour déterminer la concentration plasmatique en lanreotide.

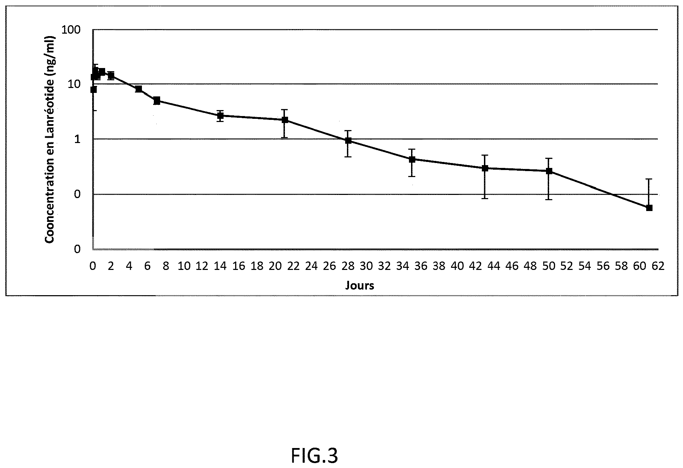

Un relargage prolongé du lanréotide est observé sur une durée d'au moins 1 mois. Les résultats sont visibles sur la Figure 3, sur laquelle est tracée l'évolution de la concentration plasmatique en lanréotide en fonction du temps.

### EXEMPLE 15

### Profil pharmacocinétique (PK) obtenu chez le chien

Une formulation préparée selon l'Exemple 1 à partir de lanréotide acétate et d'un mélange 93:7% d'eau purifiée/alcool éthylique est testée sur le chien et cela à une concentration en lanréotide de 20,7%. Des doses de 60mg de lanréotide sont injectées en intramusculaire et des échantillons de sang prélevés tout au long de l'étude. Les différents échantillons de plasma dérivés des échantillons de sang sont analysés par LC-MS pour déterminer la concentration plasmatique en lanréotide.

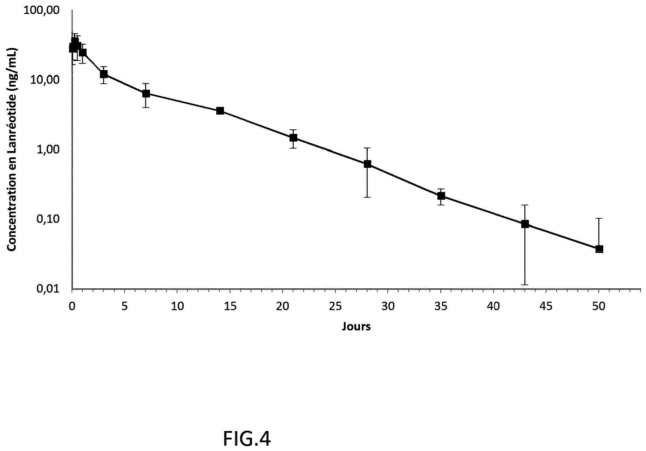

Les résultats sont visibles sur la Figure 4, sur laquelle est tracée l'évolution de la concentration plasmatique en lanréotide en fonction du temps.

Un relargage prolongé du lanréotide est observé pour une durée d'au moins 1 mois.

### EXEMPLE 16

### Préparation d'une composition aqueuse de lanréotide avec arginine et résultats analytiques de pureté

Une nouvelle composition est obtenue à partir de 0,40 g de lanréotide acétate et d'une solution de L-arginine, à 0,072g/g dans de l'eau purifiée. La composition est obtenue en mélangeant le lanréotide acétate avec 2,67g de solution d'arginine. Les 2 composants sont pesés dans des seringues plastiques de 5 mL.

Le mélange est réalisé par va-et-vient entre les 2 seringues connectées par un connecteur tel que décrit dans l'Exemple 1. A la fin de la phase de mélange la composition est répartie dans des seringues plastiques de 1,0 mL (aux alentours de 530 mg de composition par seringues). Cette composition peut être injectée telle qu'elle ou peut être lyophilisée pour obtenir une forme prête à être reconstituées avant injection.

Des analyses RP-HPLC réalisées sur les produits tels que décrites dans l'Exemple 5 indiquent la pureté du peptide est maintenue supérieure à 95% (98,6%).

### EXEMPLE 17

### Profil pharmacocinétique (PK) obtenue sur le rat avec une composition contenant de l'arginine

Une composition solide de lanréotide acétate avec de la L-arginine est testée sur le rat. La composition est préparée à partir d'une composition aqueuse à 11% de lanréotide pour obtenir au final un contenu en lanréotide de 57,9%m/m avec approximativement 0,57 g d'arginine par g de lanréotide.

Des doses d'environ 3,5mg sont injectées par voie sous-cutané et des échantillons de sang prélevés tout au long de l'étude. Les différents échantillons de plasma dérivés des échantillons de sang sont analysés par LC-MS pour déterminer la concentration plasmatique en lanréotide.

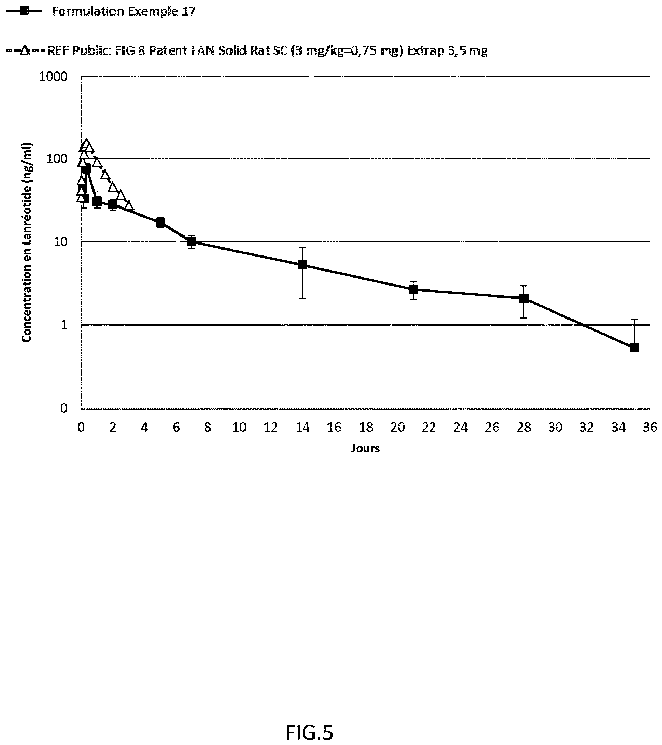

Les résultats sont visibles sur la Figure 5, sur laquelle est tracée l'évolution de la concentration plasmatique en lanréotide en fonction du temps. Un relargage prolongé de lanréotide est observé pour une durée d'au moins 4 semaines. Par comparaison, on obtient une amélioration du profil comparé au profil PK des formes équivalentes sans arginine .

### EXEMPLE 18

### Détermination de la forme de dépôts par palpation et par prélèvement extemporané de différentes compositions injectées par voie sous-cutané chez le lapin, résultats et extrapolations possibles

Des solutions à différentes concentrations sont préparées tel que décrit dans l'Exemple 1.
- Formulation A : formulation à 20% de lanréotide dans l'eau
- Formulation B : formulation à 20% de lanréotide dans un mélange eau/éthanol 95:5%
- Formulation Reference : formulation à 25% de lanréotide dans l'eau qui reproduit les conditions des produits commerciaux actuels.

Ces formulations sont injectées chez le lapin suivant le modèle suivant :
- Un premier bras est injecté à t=0 avec 30mg de Formulation A et 30 mg de Formulation Reference en deux sites d'injection différents ; à t=7 jours avec 30 mg de Formulation A à un troisième site d'injection, et à t=11 jours avec 30mg de Formulation A à un quatrième site d'injection, soit 4 injections pratiquées au total ;
- Un second bras est injecté à t=0 avec 30mg de Formulation B et 30 mg de Formulation Reference en deux sites d'injection différents ; à t=7 jours avec 30 mg de Formulation B à un troisième site d'injection, et à t=11 jours avec 30mg de Formulation B à un quatrième site d'injection, soit 4 injections pratiquées au total ;
Il est possible de percevoir à travers la peau les différents dépôts au toucher pour suivre l'évolution de leur forme et de leur consistance, et après deux semaines de vérifier leur nature par prélèvement extemporané.

On note que pour ces mêmes doses faibles de 30mg de lanréotide injectées, les dépôts préparés de Formulation Reference pourtant de volume plus faible, sont plus sensibles ou palpables que ceux des Formulations A ou B. Les dépôts moins visqueux réalisés à partir du mélange eau-alcool de la Formulation B sont les moins palpables.

Après 2 semaines au moment du sacrifice des animaux, on note que tous les dépôts à 20% de Formulation A ou Formulation B après 3, 7 ou 14 jours présentent une masse blanche plus ou moins solide qui correspond à la précipitation du lanréotide. Le contenu en lanréotide de ces dépôts est analysé, et l'on constate que les quantités restantes de ce peptide étaient inversement proportionnelles au temps après injection et comparables à 2 semaines avec celles obtenues avec le dépôt de Formulation Reference.

Cela démontre la possibilité d'obtenir avec ces nouvelles présentations qui combinent des aiguilles plus fines et des compositions plus diluées, des compositions contrôlées de lanréotide sur une semaine et plus selon un processus de relargage différent.

## Revendications

1. Seringue pré-remplie ayant un diamètre supérieur à 3,00 mm, préférentiellement compris entre 4,00 mm et 7,00 mm, et étant équipée d'une aiguille ayant une longueur comprise de préférence entre 10 et 40 mm et contenant une composition pharmaceutique comprenant :
1) un solvant aqueux constitué d'eau et éventuellement d'un solvant organique miscible à l'eau, de préférence un alcool, encore plus de préférence l'éthanol,
2) du lanréotide ou l'un de ses sels, de préférence l'acétate de lanréotide à une concentration allant de 11 à 25% en poids de lanréotide, de préférence de 18 à 25 %, en poids de lanréotide par rapport au poids total de la composition,
3) éventuellement un ou plusieurs acides aminés, de préférence l'arginine,
4) éventuellement un excipient monomérique accepté par la pharmacopée **caractérisée en ce que** ladite seringue est équipée d'une aiguille standard ayant un diamètre externe égal ou inférieur à 1,00 mm et un diamètre interne égal ou inférieur à 0,80 mm, le diamètre de ces aiguilles permettant de réduire les difficultés d'implantation des aiguilles de diamètre interne d'au moins 1,00 mm.

2. Seringue pré-remplie selon la revendication 1, **caractérisée en ce que** l'administration parentérale par injection sous cutanée, normale ou profonde, ou intramusculaire chez un patient de ladite composition à l'aide de ladite seringue libère le lanréotide ou le sel de lanréotide sur une durée de 14 à 28 jours, de préférence d'au moins 28 jours.

3. Seringue pré-remplie selon la revendication 1, **caractérisée en ce que** la composition comprend de l'éthanol dans une proportion comprise entre 3 % et 23%, de préférence de 3 à 10% en poids par rapport au poids total de la composition.

4. Seringue pré-remplie selon l'une des revendications 1 à 3, **caractérisée en ce que** la composition comprend de l'arginine, à une concentration allant de 0,1 à 13% en poids par rapport au poids total de la composition.

5. Seringue pré-remplie selon l'une des revendications 1 à 4 **caractérisée en ce que** l'aiguille standard est du type luer-lock ayant un diamètre externe de 0,80 à 1,00 mm, un diamètre interne de 0,60 à 0,80 mm et une longueur de 20 à 30 mm.

6. Seringue pré-remplie selon l'une des revendications 1 à 5 **caractérisée en ce qu'**il s'agit d'une seringue manuelle avec tige de piston munie d'une aiguille standard du type luer-lock ayant un diamètre externe de 0,80 à 1,00 mm, un diamètre interne de 0,60 à 0,80 mm et une longueur d'environ 20 mm.

7. Seringue pré-remplie selon l'une des revendications 1 à 6 **caractérisée en ce qu'**elle est pré-remplie avec une composition pharmaceutique à libération prolongée pour administration parentérale, ladite composition comprenant un solvant aqueux constitué d'eau et éventuellement d'alcool éthylique à 3%, de l'acétate de lanréotide avec une concentration en lanréotide de 18% à 25% plus préférentiellement de 18 à 22%,%, plus préférentiellement encore de 18 à 21 en poids par rapport au poids total de la composition, et optionnellement de l'arginine à une concentration allant de 0,1 à 13% en poids par rapport au poids total de la composition.

8. Seringue pré-remplie selon l'une des revendications 1 à 7 avec tige de piston munie d'une aiguille standard du type luer-lock ayant un diamètre externe d'environ 0,80 mm, un diamètre interne d'environ 0,60 mm et une longueur d'environ 20 mm, pré-remplie avec une composition pharmaceutique à libération prolongée pour administration parentérale, ladite composition comprenant de l'eau et de l'acétate de lanréotide avec une concentration en lanréotide d'environ 20% en poids par rapport à l'eau.

## Patentansprüche

1. Vorgefüllte Spritze, welche einen Durchmesser von mehr als 3,00 mm, vorzugsweise zwischen 4,00 mm und 7,00 mm, aufweist und mit einer Nadel mit einer Länge bevorzugt zwischen 10 und 40 mm ausgestattet ist und eine pharmazeutische Zusammensetzung enthält, umfassend:
1) ein wässriges Lösungsmittel, bestehend aus Wasser und gegebenenfalls aus einem organischen, mit Wasser mischbaren Lösungsmittel, bevorzugt einem Alkohol, noch mehr bevorzugt Ethanol,
2) Lanreotid oder eines seiner Salze, bevorzugt Lanreotidacetat, in einer Konzentration von 11 bis 25 %, bezogen auf das Gewicht von Lanreotid, bevorzugt von 18 bis 25 %, bezogen auf das Gewicht von Lanreotid im Verhältnis zum Gesamtgewicht der Zusammensetzung,
3) gegebenenfalls eine oder mehrere Aminosäuren, bevorzugt Arginin,
4) gegebenenfalls einen monomeren, gemäß der Pharmakopöe annehmbaren Exzipienten,
**dadurch gekennzeichnet, dass** die genannte Spritze mit einer Standard-Nadel ausgestattet ist, die einen Außendurchmesser von gleich oder kleiner als 1,00 mm und einen Innendurchmesser von gleich oder kleiner als 0,80 mm aufweist, wobei es der Durchmesser dieser Nadeln gestattet, die Implantationsschwierigkeiten von Nadeln mit einem Innendurchmesser von mindestens 1,00 mm zu reduzieren.

2. Vorgefüllte Spritze nach Anspruch 1, **dadurch gekennzeichnet, dass** die parenterale Verabreichung der genannten Zusammensetzung mit Hilfe der genannten Spritze durch normale oder tiefe subkutane oder intramuskuläre Injektion bei einem Patienten das Lanreotid oder das Lanreotidsalz über eine Dauer von 14 bis 28 Tagen, bevorzugt mindestens 28 Tagen, freisetzt.

3. Vorgefüllte Spritze nach Anspruch 1, **dadurch gekennzeichnet, dass** die Zusammensetzung Ethanol in einem Anteil zwischen 3 % und 23 %, bevorzugt von 3 bis 10 %, bezogen auf das Gewicht im Verhältnis zum Gesamtgewicht der Zusammensetzung, umfasst.

4. Vorgefüllte Spritze nach einem der Ansprüche 1 bis 3, **dadurch gekennzeichnet, dass** die Zusammensetzung Arginin in einer Konzentration von 0,1 bis 13 %, bezogen auf das Gewicht im Verhältnis zum Gesamtgewicht der Zusammensetzung, umfasst.

5. Vorgefüllte Spritze nach einem der Ansprüche 1 bis 4, **dadurch gekennzeichnet, dass** die Standard-Nadel vom Luer-Lock-Typ mit einem Außendurchmesser von 0,80 bis 1,00 mm, einem Innendurchmesser von 0,60 bis 0,80 mm und einer Länge von 20 bis 30 mm ist.

6. Vorgefüllte Spritze nach einem der Ansprüche 1 bis 5, **dadurch gekennzeichnet, dass** es sich um eine manuelle Spritze mit einem Kolbenschaft handelt, der mit einer Standard-Nadel vom Luer-Lock-Typ mit einem Außendurchmesser von 0,80 bis 1,00 mm, einem Innendurchmesser von 0,60 bis 0,80 mm und einer Länge von ungefähr 20 mm ausgestattet ist.

7. Vorgefüllte Spritze nach einem der Ansprüche 1 bis 6, **dadurch gekennzeichnet, dass** sie mit einer pharmazeutischen Zusammensetzung mit verlängerter Freisetzung zur parenteralen Verabreichung vorgefüllt ist, wobei die genannte Zusammensetzung ein wässriges Lösungsmittel, bestehend aus Wasser und gegebenenfalls aus Ethylalkohol mit 3 %, Lanreotidacetat mit einer Konzentration von Lanreotid von 18 % bis 25 %, bevorzugter von 18 bis 22 %, noch mehr bevorzugt von 18 bis 21 %, bezogen auf das Gewicht im Verhältnis zum Gesamtgewicht der Zusammensetzung, und gegebenenfalls Arginin in einer Konzentration von 0,1 bis 13 %, bezogen auf das Gewicht im Verhältnis zum Gesamtgewicht der Zusammensetzung, umfasst.

8. Vorgefüllte Spritze nach einem der Ansprüche 1 bis 7, mit einem Kolbenschaft, der mit einer Standard-Nadel vom Luer-Lock-Typ mit einem Außendurchmesser von ungefähr 0,80 mm, einem Innendurchmesser von ungefähr 0,60 mm und einer Länge von ungefähr 20 mm ausgestattet ist, welche mit einer pharmazeutischen Zusammensetzung mit verlängerter Freisetzung zur parenteralen Verabreichung vorgefüllt ist, wobei die genannte Zusammensetzung Wasser und Lanreotidacetat mit einer Konzentration von Lanreotid von ungefähr 20 %, bezogen auf das Gewicht im Verhältnis zu dem Wasser, umfasst.

## Claims

1. Pre-filled syringe having a diameter greater than 3.00 mm, preferably comprised between 4.00 mm and 7,00 mm and being equipped with a needle having a length comprised preferably between 10 and 40 mm and containing a pharmaceutical composition comprising:
1) an aqueous solvent consisting of water and optionally of an organic solvent miscible with water, preferably an alcohol, more preferably ethanol,
2) lanreotide or one of its salts, preferably lanreotide acetate at a concentration ranging from 11 to 25% by weight of lanreotide, preferably from 18 to 25% by weight of lanreotide relative to the total weight of the composition,
3) optionally one or more amino acids, preferably arginine
4) optionally a monomeric excipient accepted by the pharmacopoeia
**characterized in that** the said syringe is equipped with a standard needle having an outer diameter equal to or less than 1.00 mm and an inner diameter equal to or less than 0.80 mm, the diameter of these needles allowing to reduce the difficulty of implanting needles with an internal diameter of at least 1.00 mm.

2. Pre-filled syringe according to claim 1, **characterized in that** the parenteral administration by subcutaneous, normal or deep, or intramuscular injection into a patient of said composition using said syringe releases lanreotide or the lanreotide salt over a period of 14 to 28 days, preferably at least 28 days.

3. Pre-filled syringe according to claim 1 **characterized in that** the composition comprises ethanol in a proportion comprised between 3% and 23%, preferably from 3 to 10% by weight, relative to the total weight of the composition.

4. Pre-filled syringe according to any of claims 1 to 3 **characterized in that** the composition comprises arginine, at a concentration ranging from 0.1 to 13% by weight relative to the total weight of the composition.

5. Pre-filled syringe according to any of claims 1 to 4, **characterized in that** the standard needle is of the luer-lock type having an outer diameter of 0.80 to 1.00 mm, an inner diameter of 0.60 to 0.80 mm and a length of 20 to 30 mm.

6. Pre-filled syringe according to any of claims 1 to 5, **characterized in that** the syringe is a manual syringe with piston rod provided with a standard needle of the luer-lock type having an outer diameter of 0.80 to 1.00 mm, an inner diameter of 0.60 to 0.80 mm and a length of about 20 mm.

7. Pre-filled syringe according to any of claims 1 to 6, **characterized in that** it is pre-filled with a sustained-release pharmaceutical composition for parenteral administration, said composition comprising an aqueous solvent consisting of water and optionally 3% ethyl alcohol, acetate of lanreotide in an lanreotide concentration of 18% to 25% more preferably of 18 to 22% even more preferably of 18% to 21% by weight relative to the total weight of the composition and optionally arginine at a concentration ranging from 0.1 to 13% by weight relative to the total weight of the composition.

8. Pre-filled syringe according to any of claims 1 to 7 with a piston rod provided with a standard needle of the luer-lock type having an outer diameter of 0.80 mm, an inner diameter of 0.60 mm and a length of about 20 mm, pre-filled with a sustained release pharmaceutical composition for parenteral administration, said composition comprising water and lanreotide acetate with a lanreotide concentration of about 20% by weight relative to water.
